(19) Europäisches Patentamt
European Patent Office
Office européen des brevets

(11) EP 0 947 503 B1

(12) FASCICULE DE BREVET EUROPEEN

(45) Date de publication et mention de la délivrance du brevet:
**21.11.2001 Bulletin 2001/47**

(51) Int Cl.[7]: **C07C 327/32**, C07D 295/18, A61K 31/265

(21) Numéro de dépôt: **99400803.5**

(22) Date de dépôt: **01.04.1999**

# (54) Dérivés de l'acide 6,8-dimercaptooctanoique substitués en 6-S et/ou 8-S par le radical (3-methylthiopropanoyl) et compositions pharmaceutiques destinées au traitement de tumeurs cancéreuses

In 6-S und/oder 8-S durch 3-Methylthiopropanoylrest substituierte 6,8-Dimethylmercaptooctansäure-Derivate und pharmaceutische Zubereitungen zur Behandlung von Krebstumoren

6,8-Dimethylmercaptooctanoic acid derivatives substituted on 6-S and/or 8-S by the 3-methylthiopropanoyl radical and pharmaceutical compositions for the treatment of cancerous tumours

(84) Etats contractants désignés:
**AT BE CH CY DE DK ES FI FR GB GR IE IT LI LU MC NL PT SE**

(30) Priorité: **01.04.1998 FR 9804041**

(43) Date de publication de la demande:
**06.10.1999 Bulletin 1999/40**

(73) Titulaire: **Galderma Research & Development, S.N.C.**
**06902 Sophia Antipolis cedex (FR)**

(72) Inventeurs:
- **Quash, Gérard Anthony**
  **69340 Francheville (FR)**
- **Gore, Jacques**
  **69300 Calluire et Cuire (FR)**
- **Fournet, Guy**
  **69100 Villeurbanne (FR)**

(74) Mandataire: **Stalla-Bourdillon, Bernard**
**NONY & ASSOCIES,**
**3, rue de Penthièvre**
**75008 Paris (FR)**

(56) Documents cités:
**WO-A-96/20701**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 947 503 B1

Printed by Jouve, 75001 PARIS (FR)

## Description

La présente invention a pour objet des dérivés de l'acide 6,8-dimercaptooctanoïque substitués en 6-S et/ou 8-S par le radical (3-méthylthiopropanoyl) ainsi que leur utilisation en tant qu'agents inducteurs de l'apoptose cellulaire.

La présente invention a également pour objet une composition pharmaceutique contenant, en tant qu'agent actif, au moins un dérivé de l'acide 6,8-dimercaptooctanoïque substitué en 6-S et/ou 8-S par le radical (3-méthylthiopropanoyl), dans un excipient pharmaceutiquement acceptable, notamment pour le traitement, la régression et la prévention de tumeurs cancéreuses.

Par le terme "apoptose", on entend le phénomène de mort cellulaire tel que décrit entre autres par KERR J. F.R. et al., J. Cancer, 265, 239 (1972). L'apoptose qui est une forme hautement sélective de suicide cellulaire, se caractérise par des phénomènes morphologiques et biochimiques aisément observables. Ainsi, on observe une condensation de la chromatine associée ou non à une activité endonucléasique, la formation de corps apoptotiques et une fragmentation de l'acide désoxyribonucléique (ADN) du fait de l'activation d'endonucléases, en fragments d'ADN de 180-200 paires de base donnant un profil facilement reconnaissable par électrophorèse sur gel d'agarose.

Une très grande variété de médicaments anticancéreux naturels ou synthétiques est actuellement disponible.

Parmi ces médicaments antinéoplasiques, on peut citer les agents alkylants tels que la cyclophosphamide, les nitrosourées tels que le 1,3-bis (2-chloroéthyl)-1-nitrosourée (BCNU), les agents intercalants tels que l'actinomycine D ou l'adriamycine, les analogues de bases puriques ou pyrimidiques tels que la 6-thioguanine et la 5-fluorouracile, les inhibiteurs de la synthèse de novo des bases puriques tels que le méthotrexate et enfin les inhibiteurs de la polymérisation de la tubuline tels que le Taxol®.

Un des principaux inconvénients de l'utilisation de ces substances est l'absence d'une activité apoptotique sélective des cellules tumorales. Ainsi, le problème majeur pour la réalisation de tels composés est de concevoir et préparer des molécules qui induisent une apoptose maximale dans le tissu tumoral tout en lésant le moins possible et de façon réversible les tissus sains de l'organisme.

En vue de remédier à cette absence de sélectivité, il a déjà été proposé par la demanderesse, notamment dans la demande de brevet WO96/20701, d'utiliser un composé inducteur d'apoptose choisi parmi le méthional, le malonaldéhyde et tout facteur permettant d'augmenter le taux intracellulaire de ces composés, c'est-à-dire ayant une action sur le métabolisme du méthional représenté pour mémoire à la Figure 1 (QUASH et al., Biochem. J. 305, 1017 (1995)).

Il a également été décrit dans cette même demande de brevet, à titre de facteur augmentant le taux intracellulaire du méthional, l'utilisation d'inhibiteurs de l'acide 4-méthylthio 2-oxobutanoique (MTOB) transaminase, composés d'esters de L-méthionine et de pyridoxal. La MTOB transaminase étant une enzyme impliquée dans la conversion de l'acide 4-méthylthio 2-oxobutanoïque en méthionine, l'utilisation de ces composés favorise l'accumulation de MTOB, précurseur direct du méthional (Figure 1) (ROCH et al., Biochem. J. 313, 973 (1996)).

Toutefois, la sélectivité du méthional et du malonaldéhyde pour les cellules tumorales s'est avérée encore insuffisante puisque ces composés inhibent autant la croissance des cellules tumorales que celle des cellules normales.

Il a par ailleurs été décrit par la demanderesse dans la demande de brevet FR-9704283, l'utilisation d'un dérivé aminothioester, désigné AMPALTE, en tant qu'inhibiteur de l'enzyme aldéhyde déshydrogénase (ALDH), enzyme impliquée dans la conversion du méthional en acide méthylthiopropionique, favorisant ainsi l'accumulation de méthional. Ce composé est un inhibiteur sélectif de la croissance des cellules transformées résistantes à l'apoptose du fait de la surexpression du gène anti-apoptique $bcl_2$.

Un tel composé est donc plus spécifiquement destiné aux pathologies caractérisées par une surexpression du gène $bcl_2$ telles que les cancers du sein, les lymphomes des cellules B, les leucémies, les neuroblastomes, les adénocarcinomes de la prostate, les prolactinomas et autres adénomes pituitaires.

Il était donc souhaitable de mettre au point une stratégie thérapeutique qui soit sélective des cellules tumorales et de cibler un grand nombre de pathologies cancéreuses.

A la suite de nombreuses études sur divers composés dérivés du méthional, on a constaté que les dérivés comprenant le méthional couplé par liaison thio-ester à l'acide thioctique présentaient une forte activité apoptotique sélective pour les cellules transformées et tumorales, comme le démontrent les données expérimentales présentées dans la demande.

Une telle activité rend les composés de l'invention utiles dans le traitement thérapeutique antitumoral.

La présente invention a donc pour objet, à titre de nouveaux composés, des dérivés de l'acide 6,8-dimercaptooctanoïque substitués en 6-S et/ou 8-S par le radical (3-méthylthiopropanoyl) ainsi que leur utilisation en tant qu'agents inducteurs d'apoptose des cellules transformées et tumorales.

La présente invention a également pour objet de nouvelles compositions pharmaceutiques contenant, en tant qu'agent actif, une quantité efficace d'au moins un nouveau composé objet de l'invention dans un excipient pharmaceutiquement acceptable.

La présente invention concerne enfin de nouvelles compositions pharmaceutiques pour le traitement, la régression et la prévention du cancer contenant, en tant qu'agent thérapeutique antitumoral, une quantité efficace d'au moins un nouveau composé objet de la présente invention.

Les composés selon l'invention peuvent être représentés par la formule (I) suivante :

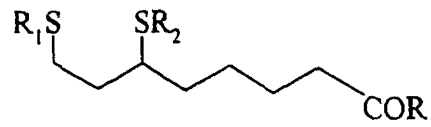
(I)

dans laquelle :

R représente $OR_3$ ou

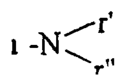

$R_3$ représentant un atome d'hydrogène, un radical alkyle, linéaire ou ramifié, ou un radical de formule

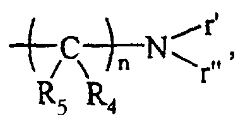

n étant un nombre entier de 1 à 10, et r' et r", identiques ou différents, représentant un atome d'hydrogène, un radical alkyle, linéaire ou ramifié ou, pris ensemble forment, avec l'atome d'azote, un hétérocycle azoté éventuellement substitué par un atome d'oxygène ou d'azote éventuellement substitué,

$R_5$ et $R_4$, identiques ou différents, représentent un atome d'hydrogène ou un radical alkyle linéaire ou ramifié,

$R_1$ et $R_2$, identiques ou différents, représentent un atome d'hydrogène, un radical $-COCH_3$ ou un radical de formule :

$$-CO(CH_2)_2SCH_3 \quad (II)$$

sous réserve que l'un au moins des radicaux $R_1$ ou $R_2$ représente un radical de formule (II) et les énantiomères R et S et leur racémique ainsi que les sels desdits composés de formule (I).

Parmi les radicaux alkyles, linéaires ou ramifiés, on préfère selon l'invention un radical ayant de 1 à 12 atomes de carbone, et plus particulièrement de 1 à 6 atomes de carbone. Parmi ceux-ci on peut citer les radicaux méthyle, éthyle, isopropyle, n-butyle iso-butyle et tertio-butyle.

Selon l'invention, l'indice "n" est généralement compris entre 2 et 6, le radical divalent étant de préférence le radical éthylène, propylène, butylène ou méthyl-2 propane-1,2 diyle.

Lorsque selon l'invention, les radicaux r' et r" forment un hétérocycle azoté, celui-ci peut être la morpholine, la pyrrolidine, la pipérazine, l'homopipérazine et une N-alkyl ($C_1$-$C_6$) pipérazine.

L'atome de carbone en position $C_6$ étant asymétrique, les composés de l'invention peuvent donc. se présenter soit sous forme des énantiomères R et S, soit sous forme racémique.

Lorsque les composés selon l'invention de formule (I) sont sous forme de sels, ceux-ci peuvent être soit des sels d'une base minérale ou organique lorsqu'ils comportent une fonction acide carboxylique, soit des sels d'un acide minéral ou organique lorsqu'ils comportent une fonction amine salifiable.

Parmi les sels d'une base on peut citer par exemple les sels de sodium, de potassium, de calcium, et parmi les sels d'un acide, les halogénures, les nitrates, les sulfates, les sulfonates, les carboxylates, les thiocyanates et les phosphates.

Selon un mode de réalisation préféré, les sels sont des halogénures d'ammonium notamment des iodures tels que ceux de trimétholammonium ou de N-alkyl pipérazonium.

Parmi les composés de formule (I) monosubstitués en position 6 ou 8 ou disubstitués on peut notamment citer :

| N° | Composés |
|---|---|
| 1- | 6-mercapto-8-S-(3-méthylthiopropanoyl) octanoate de méthyle, (formule I : $R_1$=$-CO(CH_2)_2-SCH_3$ ; $R_2$=H; R=$OCH_3$) ; |
| 2- | 6-S-(3-méthylthiopropanoyl) 8-mercapto octanoate de méthyle, (formule I : $R_1$=H; $R_2$=$-CO(CH_2)_2-SCH_3$ ; R=$OCH_3$) ; |
| 3- | le 6-S,8-S-bis (3-méthylthiopropanoyl) octanoate de méthyle, (formule I : $R_1$=$R_2$=$-CO(CH_2)_2-SCH_3$; R=$OCH_3$) ; |
| 4- | 6-mercapto-8-S-(3-méthylthiopropanoyl) octanoate de tertiobutyle, (formule I : $R_1$=$-CO(CH_2)_2-SCH_3$; $R_2$=H; R=O-tert$C_4H_9$) ; |
| 5- | 6-S-(3-méthylthiopropanoyl) 8-mercapto octanoate de tertiobutyle, (formule I : $R_1$=H; $R_2$=$-CO(CH_2)_2-SCH_3$; R=O-tert$C_4H_9$) ; |
| 6- | 6-S,8-S-bis (3-méthylthiopropanoyl) octanoate de tertiobutyle, (formule I : $R_1$=$R_2$=$-CO(CH_2)_2-SCH_3$; R=O-tert$C_4H_9$) ; |
| 7- | 6-mercapto-8-S-(3-méthylthiopropanoyl) octanoate de n-butyle, (formule I : $R_1$=$-CO(CH_2)_2-SCH_3$; $R_2$=H; R=O-n$C_4H_9$) ; |
| 8- | 6-S-(3-méthylthiopropanoyl) 8-mercapto octanoate de n-butyle, (formule I : $R_1$=H; $R_2$=$-CO(CH_2)_2-SCH_3$ ; R=O-n$C_4H_9$) ; |
| 9- | 6-S,8-S-bis (3-méthylthiopropanoyl) octanoate de n-butyle, (formule I : $R_1$=$R_2$=$-CO(CH_2)_2-SCH_3$ ; R=O-n$C_4H_9$) ; |
| 10- | acide 6-mercapto-8-S(3-méthylthiopropanoyl) octanoïque, (formule I : $R_1$=$-CO(CH_2)_2-SCH_3$; $R_2$=H; R=OH) ; |
| 11- | acide 6-S-(3-méthylthiopropanoyl) 8-mercapto octanoïque, (formule I : $R_1$=H; $R_2$=$-CO(CH_2)_2-SCH_3$; R=OH) ; |
| 12- | acide 6-S,8-S-bis(3-méthylthiopropanoyl) octanoïque, (formule I : $R_1$=$R_2$=$-CO(CH_2)_2-SCH_3$; R=OH) ; |
| 13- | 6-mercapto-8-S-(3-méthylthiopropanoyl) octanoate d'éthyle, (formule I : $R_1$=$-CO(CH_2)_2-SCH_3$; $R_2$=H; R=$OC_2H_5$) ; |
| 14- | 6-S-(3-méthylthiopropanoyl) 8-mercapto octanoate d'éthyle, (formule I : $R_1$=H; $R_2$=$-CO(CH_2)_2-SCH_3$; R=$OC_2H_5$) ; |

15- 6-S,8-S-bis (3-méthylthiopropanoyl) octanoate d'éthyle, (formule I : $R_1=R_2=-CO(CH_2)_2-SCH_3$; $R=OC_2H_5$) ;

16- 6-mercapto-8-S-(3-méthylthiopropanoyl) octanoate d'isopropyle, (formule I : $R_1=-CO(CH_2)_2-SCH_3$; $R_2=H$; $R=OCH(CH_3)_2$) ;

17- 6-S-(3-méthylthiopropanoyl) 8-mercapto octanoate d'isopropyle, (formule I : $R_1=H$; $R_2=-CO(CH_2)_2-SCH_3$; $R=OCH(CH_3)_2$) ;

18- 6-S,8-S-bis (3-méthylthiopropanoyl) octanoate d'isopropyle, (formule I : $R_1=R_2=-CO(CH_2)_2-SCH_3$; $R=OCH(CH_3)_2$) ;

19- iodure de 6-mercapto-8-S-(3-méthylthiopropanoyl) octanoate de (2' triméthyl ammonium éthyle), (formule I : $R_1=-CO(CH_2)_2-SCH_3$; $R_2=H$ ; $R=O(CH_2)_2N^+(CH_3)_3I^-$) ;

20- iodure de 6-S-(3-méthylthiopropanoyl) 8-mercapto octanoate de (2' triméthyl ammonium éthyle), (formule I : $R_1=H$; $R_2=-CO(CH_2)_2-SCH_3$; $R=O(CH_2)_2N^+(CH_3)_3I^-$) ;

21- iodure de 6-S,8-S-bis (3-méthylthiopropanoyl) octanoate de (2' triméthyl ammonium éthyle), (formule I : $R_1=R_2=-CO(CH_2)_2-SCH_3$; $R=O(CH_2)_2N+(CH_3)_3I^-$) ;

22- [6-S,8-S-bis(3-méthylthiopropanoyl)octanoyl]-N-méthyl pipérazine, (formule I : $R_1=R_2=-CO(CH_2)_2-SCH_3$ ; R= 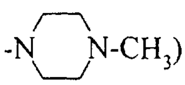 ) ;

23- 6-S-acétyl, 8-S-(3-méthylthiopropanoyl) octanoate de méthyle, (formule I : $R_1=-CO(CH_2)_2-SCH_3$ ; $R_2=-COCH_3$ ; $R=-COH_3$) ;

24- 6-S-(3-méthylthiopropanoyl), 8-S-acétyl octanoate de méthyle, (formule I : $R_1=-COCH_3$ ; $R_2=-CO(CH_3)_2-SCH_3$ ; $R=-OCH_3$) ;

25- iodure de 6-S-acétyl, 8-S-(3-méthylthiopropanoyl) octanoate de (2' triméthyl ammonium éthyle), (formule I : $R_1=-CO(CH_2)_2-SCH_3$ ; $R_2=-COCH_3$ ; $R=-O(CH_2)_2N^+(CH_3)_3I^-$) ;

26- iodure de 6-S-(3-méthylthiopropanoyl), 8-S-acétyl octanoate de (2' triméthyl ammonium éthyle), (formule I : $R_1=-COCH_3$ ; $R_2=-CO(CH_2)_2-SCH_3$ ; $R=-O(CH_2)_2N^+(CH_3)_3I^-$).

Les énantiomères (R) et (S) des composés ci-dessus seront désignés ci-après en fonction du numéro attribué à chaque composé suivi de la mention (R) ou (S), et les racémiques seront désignés en fonction du numéro suivi de la mention (RS).

Les composés de formule (I) selon l'invention peuvent être obtenus selon le schéma réactionnel de la Figure 2.

Le procédé de synthèse consiste en une première étape à estérifier l'acide 6,8-thioctique ou acide lipoïque de formule (1) soit en présence de diazométhane lorsque l'on souhaite obtenir l'ester de formule (2) dans laquelle R=$OCH_3$ soit, pour les autres significations, à l'aide d'un alcool ROH dans l'éther en présence de dicyclohexylcarbodiimide (DCC) et de 4-diméthylaminopyridine (4-DMAP). Le rendement de l'estérification est généralement d'environ 80 à 99 % par rapport à l'acide de départ.

L'ester obtenu (2) est ensuite soumis à une réduction en présence de borohydrure de sodium dans un mélange méthanol/tétrahydrofuranne pour conduire au dithiol de formule (3) avec un rendement supérieur à environ 95 %.

A partir du dithiol de formule (3), on accède aux composés de formule (Ia) et (Ib) en faisant réagir 1 équivalent molaire d'acide 3-méthylthiopropionique dans du dichlorométhane en présence de DCC et de 4-DMAP. On obtient ainsi un mélange de (Ia) et (Ib) que l'on ne peut séparer par chromatographie préparative sur gel de silice, mais dans lequel (Ia) est largement majoritaire (>90 %). Il est probable que les composés (Ia) et (Ib) soient interconvertibles.

Le composé de formule (Ic) est également obtenu à partir du dithiol de formule (3) dans les mêmes conditions que ci-dessus mais en faisant réagir un excès molaire d'acide 3-méthylthiopropionique de préférence 3 équivalents molaires. Le rendement de la réaction est d'environ 60 à 80 %.

Si, lors de la réaction, et malgré l'excès d'acide 3-méthylthiopropionique, il se forme l'un des composés des formules (Ia) et/ou (Ib), une chromatographie sur gel de silice permet de les séparer.

Les composés de formule (I) (R=OH) peuvent être obtenus directement à partir de l'acide lipoïque par réduction et acylation à l'aide de l'acide 3-méthylthiopropionique.

Les amides de formule (I) sont aisément accessibles à partir de l'acide thioctique par la même séquence à savoir amidation, réduction et acylation.

Bien qu'il ait été fait référence ci-dessus à certaines conditions particulières des étapes de synthèse des composés selon l'invention, il va de soi que d'autres variantes de ces conditions peuvent être utilisées notamment en ce qui concerne les étapes d'estérification ou d'amidation et d'acylation.

La présente invention a également pour objet une composition pharmaceutique contenant, en une quantité efficace, au moins un dérivé de l'acide 6,8-dimercaptooctanoïque substitué en 6-S et/ou 8-S par le radical (3-méthylthiopropanoyl) de formule (I) en tant qu'agent actif, plus particulièrement en tant qu'agent inducteur d'apoptose des cellules transformées et tumorales, dans un excipient pharmaceutiquement acceptable.

La composition pharmaceutique selon la présente invention induit les phénomènes d'apoptose tels que définis ci-dessus, de manière sélective dans les cellules transformées et cancéreuses.

Plus particulièrement, la présente invention a pour objet une composition pharmaceutique pour le traitement, la régression et la prévention de tumeurs cancéreuses chez un sujet humain ou animal.

La composition pharmaceutique selon l'invention comprend un milieu physiologiquement acceptable et peut être administrée par voie entérale, parentérale, ou topique.

De préférence, la composition pharmaceutique est conditionnée sous une forme convenant à une application par voie systémique (pour injection ou perfusion).

Par voie entérale, la composition pharmaceutique peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée.

Par voie parentérale, la composition peut se présenter sous forme de solutions ou suspensions pour perfusion ou pour injection.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions.

Elle peut également se présenter sous forme de microsphères ou nanosphères ou vésicules lipidiques ou polymériques ou de patchs polymériques et d'hydrogels permettant une libération contrôlée. Lorsque la composition est destinée à un usage par voie topique, elle peut se présenter soit sous forme anhydre, soit sous forme aqueuse.

Dans les compositions selon l'invention, le composé actif de formule (I) est généralement présent en une concentration comprise entre 0,1 et 10% en poids, de préférence entre 0,1 et 5 % en poids, par rapport au poids total de la composition.

Les compositions telles que décrites précédemment peuvent bien entendu contenir en outre des additifs inertes ou même pharmacodynamiquement actifs ou des combinaisons de ces additifs, et notamment d'autres agents antinéoplasiques, tels que par exemple la dexaméthasone, la cyclophosphamide, le cisplatine, l'étoposide et le BCNU (N,N-Bis(2-chloroéthyl)-N-nitrosourée), qui sont également capables d'induire l'apoptose.

Les composés actifs de formule (I) selon l'invention sont généralement administrés à une dose journalière

d'environ 50 mg/kg à 100 mg/kg de poids corporel en 1 à 2 prises. La durée du traitement étant fonction des états pathologiques des sujets.

Les différents aspects de l'invention apparaîtront plus clairement à la lecture des divers exemples suivants destinés à l'illustrer.

## EXEMPLES

**Exemple 1** : *Préparation du 6-mercapto-8-S-(3-méthylthiopropanoyl) octanoate de méthyle (composé N°1)*

(a) *6,8-dimercapto octanoate de méthyle*

Le lipoate de méthyle de départ est un composé connu qui a été décrit par Gunsalus et al., J.Am.Chem.Soc. 1956, 78, 1763-1766.

A 444 mg (2,02 mmol) de lipoate de méthyle en solution dans 25 ml d'un mélange méthanol/tétrahydrofuranne (5/1), on ajoute à 0°C en deux fois 85 mg (2,25 mmol) de borohydrure de sodium.

Après 40 mn d'agitation à 0°C, on verse goutte à goutte de l'acide chlorhydrique 5N jusqu'à l'apparition d'un trouble blanc stable. Après évaporation du solvant, le résidu est repris dans de l'éther et on lave la phase organique par une solution aqueuse saturée de bicarbonate de sodium puis par une solution saturée de chlorure de sodium. Après séchage sur sulfate de sodium et évaporation de l'éther, on obtient 445 mg (99 %) du dithiol attendu.

(b) *6-mercapto-8-S-(3-méthylthiopropanoyl) octanoate de méthyle*

A 192 mg (1,6 mmol) d'acide 3-méthylthiopropanoïque dans 0,6 ml de dichlorométhane, on ajoute 8 mg (0,065 mmol) de 4-diméthylaminopyridine puis on introduit une solution de 355 mg (1,6 mmol) de 6,8-dimercaptooctanoate de méthyle, obtenue ci-dessus, dans 1 ml de dichlorométhane. On ajoute ensuite 33 mg (1,6 mmol) de dicyclohexylcarbodiimide à 0°C et laisse 5 mn à 0°C. Le mélange est ensuite porté à 18°C sous agitation pendant 3 heures. Après filtration et dilution du filtrat dans l'éther, on lave la phase organique avec une solution aqueuse saturée de chlorure de sodium, on sèche sur sulfate de magnésium et évapore l'éther. Le résidu obtenu est alors purifié par chromatographie sur gel de silice (25 g, éluant : éther de pétrole/acétate d'éthyle 85/15) et on isole 311 mg (60 %) du mercaptoester attendu sous forme d'une huile presque incolore (contaminant 5 à 10 % de 6-S-(3-méthylthiopropanoyl) 8-mercapto octanoate de méthyle).

**Exemple 2 :** *Préparation du 6-S,8-S-bis (3-méthylthiopanoyl) octanoate de méthyle (composé N°3)*

Ce composé est obtenu selon le même mode opératoire que celui décrit à l'exemple 1(b) mais en utilisant 3 équivalents molaires d'acide 3-méthylthiopropanoïque.

Le produit attendu est obtenu avec un rendement de 75 % sous forme d'une huile presque incolore.

$$[\alpha]_D = -6,1 \text{ (c:1,93 } CHCl_3\text{) Série R}$$

$$[\alpha]_D = +6,8 \text{ (c:2 } CHCl_3\text{) Série S}$$

**Exemple 3** : *Préparation du 6-mercapto-8-S-(3-méthylthiopropanoyl) octanoate d'isopropyle (composé N°16)*

(a) *Lipoate d'isopropyle*

A 1 g (4,85mmol) d'acide lipoïque, 1,1 g (5,33 mmol) de dicyclohexylcarbodiimide et 0,061 g (0,5 mmol) de 4-diméthylaminopyridine placés sous azote, on ajoute 12 ml d'éther anhydre. Après 10 mn d'agitation à température ambiante, on ajoute 0,41 ml (5,35 mmol) d'isopropanol et on agite pendant 15 heures.

Après filtration des solides et dilution du filtrat dans l'éther, on lave la phase organique par une solution d'acide acétique à 5 % dans l'eau, puis par une solution aqueuse saturée de bicarbonate de sodium et enfin par une solution aqueuse saturée de chlorure de sodium.

Après séchage sur sulfate de sodium et évaporation du solvant, le résidu est filtré sur gel de silice (20 g, éluant : éther de pétrole/acétate d'éthyle 85/15).

Après évaporation du solvant d'élution, on obtient 1,095 g (91 %) de lipoate d'isopropyle sous forme d'une huile jaune.

(b) *6,8-dimercapto octanoate d'isopropyle*

Ce composé est obtenu selon le même mode opératoire que celui décrit à l'exemple 1(a) par réduction du lipoate d'isopropyle à l'aide de borohydrure de sodium.
Echelle: 2mmol, rendement : 99 % (huile presque incolore).

(c) *6-mercapto-8-S-(3-méthylthiopropanoyl)octanoate d'isopropyle*

Ce composé est obtenu selon le même mode opératoire que celui décrit à l'exemple 1(b) par acylation du 6,8-dimercapto octanoate d'isopropyle à l'aide d'un équivalent molaire d'acide 3-méthylthio-propanoïque. Après purification par chromatographie sur gel de silice, on isole le 6-mercapto-8-S-(3-méthylthiopropanoyl) octanoate d'isopropyle sous forme d'une huile (rendement : 60 %) (contaminant: 5 à 10 % de 6-S-(3-méthylthiopropanoyl) 8-mercapto octanoate d'isopropyle et le 6-S, 8-S-bis(3méthylthiopropanyl) octanoate d'isopropyle. Echelle : 2 mmol, rendement : 11 %.

**Exemple 4** : *Préparation du 6-S,8-S-bis (3-méthylthiopropanoyl) octanoate de n-butyle (composé N° 9)*

(a) *Lipoate de n-butyle*

Selon le même mode opératoire que celui décrit à l'exemple 3(a) pour la synthèse du lipoate d'isopropyle, l'estérification de l'acide lipoïque à l'aide de n-butanol conduit au produit attendu avec un rendement de 78 % sous forme d'une huile presque incolore. Echelle : 5 mmol.

(b) *6,8-dimercapto octanoate de n-butyle*

Selon le même mode opératoire que celui décrit à l'exemple 1(a) pour la synthèse du 6,8-dimercapto octanoate de méthyle, la réduction du lipoate de n-butyle à l'aide du borohydrure de sodium conduit au produit attendu avec un rendement de 95 % sous forme d'une huile presque incolore. Echelle : 2 mmol.

(c) *6-S,8-S-bis (3-méthylthiopropanoyl) octanoate de n-butyle*

Selon le même mode opératoire que celui décrit à l'exemple 2 pour la synthèse du 6-S,8-S-bis (3-méthylthiopropanoyl) octanoate de méthyle, l'acylation du 6,8-dimercapto octanoate de n-butyle à l'aide de 3 équivalents molaires d'acide 3-méthylthiopropanoïque conduit au produit attendu avec un rendement de 86 % sous forme d'une huile presque incolore. Echelle : 4 mmol.

**Exemple 5** : *Préparation du 6-mercapto-8-S-(3-méthylthiopropanyl) octanoate de tert-butyle (composé N° 4)*

(a) *lipoate de t-butyle*

Le mode opératoire est identique à celui décrit à l'exemple 3(a) pour la synthèse du lipoate d'isopropyle. Par estérification de l'acide lipoïque à l'aide du tert-butanol, on obtient le produit attendu avec un rendement de 40 % sous forme d'une huile presque incolore. Echelle : 11 mmol.

(b) *6,8-dimercapto octanoate de t-butyle*

Selon le même mode opératoire que celui décrit à l'exemple 1(a) pour la synthèse du 6,8-dimercapto octanoate de méthyle, la réduction du lipoate de tert-butyle à l'aide du borohydrure de sodium conduit au produit attendu avec un rendement de 95 % sous forme d'une huile presque incolore. Echelle : 11 mmol.

(c) *6-mercapto-8-S-(3-méthylthiopropanoyl) octanoate de t-butyle*

Le mode opératoire est identique à celui décrit à l'exemple 1(a) pour la synthèse du 6-mercapto-8-S-(3-méthylthiopropanoyl) octanoate de méthyle. Par acylation du 6,8-dimercapto octanoate de t-butyle à l'aide d'un équivalent molaire d'acide 3-méthylthiopropanoïque, on obtient le produit attendu avec un rendement de 60 % sous forme d'une huile presque incolore (contaminant: 5% à 10 % de 6-S-(3-méthylthiopropanoyl) 8-mercapto octanoate de t-butyle). Echelle : 10 mmol.

**Exemple 6 :** *Préparation du 6-S,8-S-bis (3-méthylthiopropanoyl) octanoate de t-butyle (composé N°6)*

Ce composé est préparé selon le même mode opératoire que celui décrit à l'exemple 2 pour la synthèse du 6-S,8-S-bis (3-méthylthiopropanoyl) octanoate de méthyle. L'acylation du 6,8-dimercapto octanoate de tert-butyle à l'aide de 3 équivalents molaires d'acide 3-méthylthiopropanoïque conduit au produit attendu avec un rendement de 77% sous forme d'une huile presque incolore. Echelle : 1 mmol.

**Exemple 7 :** *Préparation de l'iodure de 6-S,8-S-bis (3-méthylthiopropanoyl) octanoate de (2'-triméthylammonium éthyle) (composé N°21)*

*(a) lipoate de 2-diméthylamino éthyle*

A 1 g (4,85 mmol) d'acide lipoïque, 1,1 g (5,33 mmol) de dicyclohexylcarbodiimide et 0,061 g (0,5 mmol) de 4-diméthylaminopyridine placés sous azote, on ajoute 12 ml d'éther anhydre. Après 10 minutes d'agitation à température ambiante, 0,506 ml (5,33 mmol) de 2-diméthylamino éthanol est ajouté en une seule fois et on agite pendant 18 heures. Après filtration des solides et dilution du filtrat dans le dichlorométhane, on lave par une solution aqueuse saturée de chlorure de sodium. Après séchage sur sulfate de sodium et évaporation du solvant, le produit brut est purifié par chromatographie sur gel de silice (80g, éluant : $CH_2Cl_2$:MeOH = 90/10). On isole ainsi 832 mg (62 %) de l'ester attendu sous forme d'une huile jaune.

*(b) 6,8-dimercapto octanoate de 2-diméthylamino éthyle*

A 820 mg (3,0 mmol) de lipoate de 2-diméthylamino éthyle obtenu ci-dessus en solution dans 45 ml d'un mélange 5/1 méthanol/tétrahydrofuranne, on ajoute, à 0°C, 134 mg (3,6 mmol) de borohydrure de sodium en deux fois. Après 3 heures d'agitation à 0°C, on ajoute goutte à goutte HCl 1N jusqu'à pH 5 puis on évapore le solvant, et le résidu est repris dans le dichlorométhane. On lave par une solution aqueuse saturée de bicarbonate de sodium puis par une solution aqueuse saturée de chlorure de sodium. Après séchage sur sulfate de sodium et évaporation, on obtient 780 mg (93 %) du dithiol attendu sous forme d'une huile presque incolore.

*(c) 6-S,8-S-bis (3-méthylthiopropanoyl) octanoate de 2-diméthylamino éthyle*

A 233 mg (0,84 mmol) de 6,8-dimercapto octanoate de 2-diméthylamino éthyle en solution dans 1,5 ml de dichlorométhane, on ajoute 1,2 ml de pyridine et on introduit ensuite à 0°C une solution de 348 mg (2,5 mmol) de chlorure de 3-méthylthiopropanoyle dans 2,7 ml de dichlorométhane. On porte à la température ambiante et agite pendant 18 heures. Le milieu réactionnel est partagé entre une solution aqueuse saturée de bicarbonate de sodium et de dichlorométhane. La phase aqueuse est alors extraite 3 fois au dichlorométhane et l'on sèche la phase organique sur sulfate de sodium. Après évaporation, le produit brut est purifié par chromatographie sur gel de silice (40g, éluant : $CH_2Cl_2$ : MeOH = 96/4). On isole ainsi 242 mg (60 %) du produit attendu sous forme d'une huile.

(d) *iodure de 6-S,8-S-bis (3-méthylthiopropanoyl) octanoate de (2'-triméthyl ammonium éthyle)*

A 250 mg (0,52 mmol) de 6-S,8-S-bis (3-méthylthiopropanoyl) octanoate de 2-diméthylamino éthyle dans 5,2 ml d'acétate d'éthyle, on ajoute 0,048 ml (0,77 mmol) d'iodure de méthyle à température ambiante puis on poursuit l'agitation pendant 5 heures. Le solvant est alors évaporé sous vide et le résidu est repris par 5 ml d'éther anhydre et la phase éthérée est éliminée. Le produit brut est purifié par chromatographie sur gel de silice (5g, éluant: $CH_2Cl_2$: MeOH = 90/100). On isole 264 mg (81 %) du sel d'ammonium attendu sous forme non cristallisée et hygroscopique.

**Exemple 8** : *Préparation de la [6-S,8-S-bis (3-méthylthiopropanoyl) octanoyl] N-méthylpipérazine (composé N°22)*

(a) *(lipoyl)-N-méthyl pipérazine*

A 200 mg (0,97 mmol) d'acide lipoïque, 240 mg (1,16 mmol) de dicyclohexylcarbodiimide et 12 mg (0,1 mmol) de 4-diméthylaminopyridine dans 1 ml de dichlorométhane, on ajoute à température ambiante une solution de 107 mg (1,07 mmol) de N-méthyl pipérazine dans 1,2 ml de dichlorométhane. Après 23 heures d'agitation, filtration des solides et dilution du filtrat dans le dichlorométhane, on lave par une solution aqueuse saturée de chlorure de sodium. Après séchage sur sulfate de sodium et évaporation du solvant, le produit brut est purifié par chromatographie sur gel de silice (22 g, éluant : $CH_2Cl_2$:MeOH = 92/8). On isole 216 mg (77 %) d'amide attendu sous forme d'une huile jaune.

(b) *(6,8-dimercapto octanoyl)-N-méthyl pipérazine*

Ce composé est obtenu selon le même mode opératoire que celui décrit à l'exemple 7(b) par réduction du (lipoyl)-N-méthyl pipérazine à l'aide du borohydrure de sodium. On obtient le produit attendu avec un rendement de 94 % sous forme d'une huile presque incolore. Echelle : 0,4 mmol.

(c) *[6-S,8-S-bis (3-méthylthiopropanoyl) octanoyl]-N-méthyl pipérazine*

Le mode opératoire est identique à celui décrit à l'exemple 7(c) pour la synthèse du 6-S,8-S-bis (3-méthylthiopropanoyl) octanoate de 2-diméthylamino éthyle. L'acylation du (6,8-dimercapto octanoyl) N-méthyl pipérazine à l'aide de 3 équivalents molaires de chlorure de 3-méthylthiopropanoyle conduit au produit attendu avec un rendement de 90 % sous forme d'une huile presque incolore. Echelle : 0,4 mmol.

**Exemple 9** : *Préparation du 6-S-(3-méthylthiopropanoyl), 8-S-acétyl octanoate de méthyle (composé N° 24)*

(a) *6-mercapto, 8-S-acétyl octanoate de méthyle*

A un mélange de 3,045 g (13,7 mmol) de 6,8-dimercapto octanoate de méthyle et 11,08 ml (13,7 mmol) de pyridine sont ajoutés goutte à goutte à 0°C 1,29 ml (13,7 mmol) d'anhydride acétique, le mélange est ensuite porté à température ambiante et agité pendant 20 h.

Après dilution dans l'éther et lavages par une solution d'acide chlorhydrique 1N et par une solution aqueuse saturée de chlorure de sodium, on sèche sur sulfate de sodium. Après évaporation du solvant, le produit brut est purifié par chromatographie sur gel de silice (200 g éther de pétrole / acétate d'éthyle = 90/10). On isole ainsi 2,235 g (61 %) de monoacétate attendu sous forme d'une huile légèrement jaune (contaminant : 6-S-acétyl, 8-mercapto octanoate de méthyle : environ 10 %).

(b) *6-S-(3-méthylthiopropanoyl) , 8-S-acétyl octanoate de méthyle*

A 2,23 g (8,4mmol) de 6-mercapto, 8-S-acétyl octanoate de méthyle en solution dans 35 ml de dichlorométhane est ajouté 1,76 ml (10,1 mmol) de diisopropyléthylamine. Une solution de 1,403 g (10,1 mmol) de chlorure de 3-méthylthiopropanoyle dans 5 ml de dichlorométhane est ajouté à -50°C et le milieu réactionnel est porté à -25°C en 30 mn.

Après dilution dans l'éther et lavages par une solution aqueuse saturée de chlorure de sodium, on sèche sur sulfate de sodium. Après évaporation du solvant, le produit brut est purifié par chromatographie sur gel de silice (200 g éther de pétrole / acétate d'éthyle = 90/10). On isole ainsi 2,96 g (96 %) de produit attendu (contaminant : 6-S-acétyl, 8-S-(3-méthylthiopropanoyl) octanoate de méthyle : environ 10 %).

**Exemple 10 :** *Préparation du 6-S-acétyl, 8-S-(3-méthylthiopropanoyl) octanoate de méthyle (composé N° 23)*

(a) *Anhydride de l'acide 3-méthylthiopropanoïque*

A 0,345 ml (3,33 mmol) d'acide 3-méthylthiopropanoïque en solution dans 9 ml d'éther, sont ajoutés 378 mg (1,83 mmol) de dicyclohexylcarbodiimide. Après 24 h de réaction à température ambiante, la dicyclohexylurée est éliminée par filtration et le filtrat est évaporé à sec. On obtient ainsi l'anhydride attendu (rdt. Quantitatif) que l'on utilise immédiatement dans l'étape suivante.

(b) *6-mercapto, 8-S-(3-méthylthiopropanoyl) octanoate de méthyle*

Ce composé est obtenu selon le même mode opératoire que celui décrit à l'exemple 9(a) pour la synthèse du 6-mercapto, 8-S-acétyl octanoate de méthyle en remplaçant l'anhydride acétique par l'anhydride de l'acide 3-méthylthiopropanoïque décrit ci-dessus en (a). Le produit attendu est obtenu avec un rendement de 60 % (contaminant : environ 10 % de 6-S-(3-méthylthiopropanoyl), 8-mercapto octanoate de méthyle).

Echelle : 1,7 mmol

Purification par chromatographie sur gel de silice (60g éther de pétrole / acétate d'éthyle = 90/10).

(c) *6-S-acétyl, 8-S-(3-méthylthiopropanoyl) octanoate de méthyle*

Le mode opératoire est identique à celui décrit à l'exemple 9(a) pour la synthèse du 6-mercapto, 8-S-acétyl

octanoate de méthyle (acétylation à l'aide d'anhydride acétique dans la pyridine). Le produit attendu est obtenu avec un rendement de 88 % (contaminant: environ 10% de 6-S-(3-méthylthiopropanoyl), 8-S-acétyl octanoate de méthyle).

Echelle : 0,4 mmol

Purification par chromatographie sur gel de silice (5 g éther de pétrole / acétate d'éthyle = 85/15).

ETUDES D'ACTIVITE

**I**- *Effet des composés N° 1(RS), 1(S), 1(R), 3(RS), 4(RS), 6(RS), 9(RS), 21(RS) et 24(RS) sur la croissance de lignées cellulaires normales et transformées*

L'activité d'inhibition de croissance des composés de l'invention est établie sur la lignée semi-continue de fibroblastes pulmonaires humains MRC-5 d'origine embryonnaire, et soit sur les lignées cellulaires transformées HeLa établies à partir de cellules épithéliales humaines issues d'un cancer utérin, soit les lignées DU 145, PC3 et LNCaP établies à partir de cancers de la prostate et sur les lignées B16 de mélanomes de souris.

Les différentes lignées cellulaires sont étalées sur boite de Pétri à une concentration de $0,5x10^6$ et sont cultivées dans 3 ml d'un milieu essentiel de Eagle contenant 10 % en volume de sérum de veau foetal dialysé.

Après 4 heures, les cellules sont mises en culture en présence des différents composés à tester en concentration croissante (1 à 800 μM). Après incubation pendant 72 heures, les cellules sont lavées deux fois dans un milieu salin tamponné à pH 7,5 et phosphaté (tampon PBS) puis récupérées dans le même tampon PBS.

L'inhibition de la croissance cellulaire est évaluée soit en mesurant la teneur en protéines selon la méthode décrite par Lowry et al. (J. Biol. Chem., 193, 265, 1951), soit en mesurant la teneur en ADN à l'aide du colorant de Hoechst selon la méthode décrite par West et al. (Analytical Biochem. 147, 289 (1985), ou encore en déterminant l'activité des déshydrogénases selon la méthode décrite par Mosmann T. (J. Immunol. Methods, 65, 55, 1983).

Les résultats des tests d'inhibition sélective de la croissance cellulaire pour les composés N°1 (RS), 1(R), 1 (S), 3 (RS), 4 (RS), 6 (RS), 9 (RS), 21 (RS) et 24(RS) testés sont rassemblés dans les Figures 3 à 11.

Les résultats rassemblés à la Figure 3 montrent clairement que le composé N°1(RS) induit une inhibition de croissance des cellules HeLa de 40% à une concentration de 400 μM et de 70 % à une concentration de 600 μM tandis que l'inhibition de croissance des cellules fibroblastiques normales MRC-5 ne dépasse pas 5-10% aux mêmes concentrations.

Le composé N°1(S) (voir Figure 4) inhibe également de manière sélective la croissance des cellules HeLa avec une inhibition s'élevant à 90 % à une concentration de 400 μM et de 100 % à une concentration de 600 μM, alors que l'inhibition de croissance des cellules normales MRC-5 ne dépasse pas 20 % même à des concentrations plus élevées comprises entre 600 et 800 μM.

Enfin, le composé N°1 (R) (voir Figure 5) inhibe la croissance des cellules HeLa de 40 % à une concentration de 400 μM, et de 100 % à des concentrations comprises entre 600 et 800 μM.

Le composé N°1, soit sous sa forme racémique, soit sous forme de l'un de ses énantiomères, est capable d'inhiber, à des concentrations comprises entre 400 et 600 μM, de manière sélective et efficace, la croissance de cellules transformées HeLa en n'inhibant que faiblement celle des cellules fibroblastiques normales MRC-5.

Par ailleurs, les résultats obtenus avec le composé N°3 (RS) sont rassemblés à la Figure 6, et montrent qu'à des concentrations comprises entre 200 et 600 μM, celui-ci induit une inhibition de croissance des cellules HeLa de 50 % et n'inhibe pas la croissance des cellules normales MRC-5.

Selon les résultats illustrés à la Figure 7, le composé N°4 (RS) est un inhibiteur très sélectif de la croissance des cellules HeLa, puisque celui-ci induit une inhibition de croissance sélective des cellules HeLa d'environ 70 %, à partir d'une concentration de 200 μM, tandis qu'il n'inhibe la croissance des cellules MRC-5 que de 20 % à une concentration équivalente.

Le composé N°6 sous sa forme racémique (Figure 8) est capable d'inhiber à des concentrations comprises entre 50 et 600 μM de manière sélective et efficace la croissance de cellules transformées HeLa. En revanche, la croissance de cellules normales MRC-5 est stimulée de l'ordre de 40 %.

Le composé N°9 sous sa forme racémique (Figure 9) est capable d'inhiber à des concentrations comprises entre 50 et 600 μM de manière sélective et efficace la croissance de cellules transformées HeLa. En revanche, la croissance de cellules normales MRC-5 est stimulée de l'ordre de 20 %.

Les résultats présentés à la Figure 10 montrent que le composé 21 sous sa forme racémique est capable d'inhiber la croissance de cellules transformées HeLa de 80 % à 400 μM tandis que celle de cellules normales n'est inhibée que de 20 % à cette même concentration.

Des résultats similaires ont été observés à l'égard des stéréoisomères R et S des composés 6, 9 et 21.

Enfin, les résultats rassemblés à la Figure 11 montrent que le composé N°24 sous sa forme racémique est capable d'induire une inhibition de croissance des cellules transformées murines B16 et humaines DU 145, PC3 et LNCaP de 50 à 60 % à une concentration de 400 μM alors qu'à cette même concentration, l'inhibition des cellules

normales MRC-S est seulement de 10 %.

Cette inhibition de croissance des cellules transformées telles que les cellules HeLa est due à l'augmentation du pourcentage de cellules entrant en apoptose, comme cela sera démontré ci-après. En effet, le pourcentage de fragmentation de l'ADN est corrélé avec le phénomène d'apoptose.

**II :** *Effet du composé N°1 (RS) en tant qu'agent inducteur de l'apoptose mesurée par la fragmentation de l'ADN dans les cellules HeLa*

L'analyse quantitative des fragments d'ADN de taille inférieure à 3 Kb a été réalisée selon la méthode décrite par Wright S. et al. (J. Cell. Biochem., 48, 344-355), en incubant $2{,}5\times10^5$ cellules HeLa/ml avec 0.5µCi[3H]-thymidine pendant 40 heures à 37°C. Après deux lavages avec le milieu de culture, les cellules sont mises en culture en présence du composé N°1 (RS) à tester à une concentration de 200 µM. Après incubation pendant 6 heures, les cellules sont récupérées par centrifugation à 400g pendant 5 minutes et lavées 3 fois dans du tampon PBS. Les cellules récupérées dans le culot sont lysées dans 2 ml à 0,1 % de Triton X-100, 20 mM d'EDTA, 5 mM Tris pH 8 et centrifugées à 30000 g à 4°C pendant 30 minutes. Les surnageants sont récupérés et les culots dissous dans 0,3ml de 0,5N NaOH. Des aliquots du milieu de culture (1ml), du surnageant (0,3ml), et du culot solubilisé (0,1ml) sont mis à doser dans un compteur à scintillation.

Le pourcentage de fragments d'ADN est calculé de la façon suivante:

$$\text{\% de fragments} = \frac{\text{dpm du milieu de culture + dpm du surnageant d'ADN}}{\text{dpm du milieu de culture + dpm du surnageant + dpm du culot solubilisé}}$$

Les résultats qui sont rassemblés dans la Figure 12 montrent très clairement que, dans les cellules HeLa, le composé N°1 (RS) induit une augmentation du pourcentage de fragmentation de l'ADN de l'ordre de 17 % à 100 µM et 30 % à 200 µM.

**III :** *Action antitumorale in vivo du composé N°1 (RS)*

L'action antitumorale du composé N°1 (RS) a été testée *in vivo* sur des souris B6D2F1 (IFFA CREDO, France) qui ont reçu à t=0 jour, $2\times10^5$ cellules de mélanomes murins B16 en injection sous-cutanée.

A t=1 jour et ceci de manière continue une fois par jour pendant 15 jours, les souris ont été injectées par voie intrapéritonéale avec les régimes thérapeutiques suivants : éthanol 10 % dans une solution aqueuse de NaCl 0,14M, et le composé N°1(RS) soit à 50 mg/kg, soit à 100 mg/kg.

La croissance de la tumeur est ensuite observée sur une période allant de 10 à 60 jours. Les résultats sont exprimés en pourcentage de souris présentant des tumeurs.

La Figure 13 montre que le composé N°1(RS) est très efficace pour contrôler la croissance de la tumeur. En effet, le pourcentage de souris qui développent des tumeurs atteint 50 % dans les contrôles qui ont reçu le véhicule constitué de 10 % d'éthanol dans une solution aqueuse de NaCl 0,14 M, tandis qu'il n'atteint que 20 % et 10% chez les souris ayant reçu le composé 1 (RS) à 50 mg/kg et 100 mg/kg, respectivement.

LEGENDE DES FIGURES

**Figure 1** représente la voie métabolique du méthional.

**Figure 2** représente le schéma réactionnel d'obtention des composés de formule (I).

**Figure 3** représente les courbes d'inhibition de croissance des cellules HeLa (-●-) et des cellules MRC-5 (-O-) après incubation avec le composé N°1(RS).

**Figure 4** représente les courbes d'inhibition de croissance des cellules HeLa (-●-) et des cellules MRC-5 (-O-) après incubation avec le composé N°1(S).

**Figure 5** représente les courbes d'inhibition de croissance des cellules HeLa (-●-) et des cellules MRC-5 (-O-) après incubation avec le composé N°1(R).

**Figure 6** représente les courbes d'inhibition de croissance des cellules HeLa (-●-) et des cellules MRC-5 (-O-)

après incubation avec le racémique du composé N°3(RS).

**Figure 7** représente les courbes d'inhibition de croissance des cellules HeLa (-●-) et des cellules MRC-5 (-O-) après incubation avec le composé N°4(RS).

**Figure 8** représente les courbes d'inhibition de croissance des cellules HeLa (-●-) et des cellules MRC-5 (-O-) après incubation avec le composé N°6(RS).

**Figure 9** représente les courbes d'inhibition de croissance des cellules HeLa (-●-) et des cellules MRC-5 (-○-) après incubation avec le composé N°9(RS).

**Figure 10** représente les courbes d'inhibition de croissance des cellules HeLa (-●-) et des cellules MRC-5 (-O-) après incubation avec le composé N°21(RS).

**Figure 11** représente les courbes d'inhibition de croissance des cellules B16 (-●-), DU 145 (-∆-) , PC3 (-▲-), LNCaP (-□-) et des cellules MRC-5 (-O-) après incubation avec le composé N°24(RS).

**Figure 12** illustre la quantification de la fragmentation de l'ADN dans les cellules HeLa après incubation avec 200 µM du composé N°1(RS) par radiomarquage des fragments d'ADN.

**Figure 13** Représente la croissance tumorale chez des souris qui ont reçu pendant 15 jours par injection de l'éthanol 10 % dans 0,14M de NaCl (-○-) ; le composé N°1(RS) à 50mg/kg (-▲-), à 100 mg/kg (-□-).

## Revendications

1. Dérivés de l'acide 6,8-dimercaptooctanoïque substitués en 6-S et/ou 8-S par le radical (3-méthylthiopropanoyl) **caractérisés par le fait qu'**ils répondent à la formule (I) suivante :

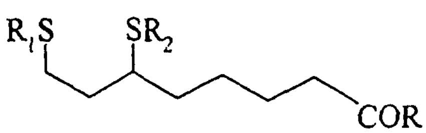

(I)

dans laquelle :

R représente $OR_3$ ou

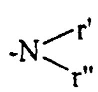

$R_3$ représentant un atome d'hydrogène, un radical alkyle, linéaire ou ramifié, ou un radical de formule

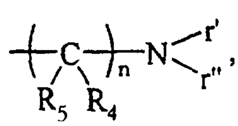

n étant un nombre entier de 1 à 10, et r' et r", identiques ou différents, représentant un atome d'hydrogène, un radical alkyle, linéaire ou ramifié ou, pris ensemble forment, avec l'atome d'azote, un hétérocycle azoté éventuellement substitué par un atome d'oxygène ou d'azote éventuellement substitué,
$R_5$ et $R_4$, identiques ou différents, représentent un atome d'hydrogène ou un radical alkyle linéaire ou ramifié,
$R_1$ et $R_2$, identiques ou différents, représentent un atome d'hydrogène, un radical $-COCH_3$ ou un radical de formule :

$$-CO(CH_2)_2SCH_3 \quad (II)$$

sous réserve que l'un au moins des radicaux $R_1$ ou $R_2$ représente un radical de formule (II) et les énantiomères R et S et leur racémique ainsi que les sels desdits composés de formule (I).

2. Composés selon la revendication 1, **caractérisés par le fait que** le radical alkyle, linéaire ou ramifié, contient de 1 à 6 atomes de carbone.

3. Composés selon la revendication 1, **caractérisé par le fait que** l'indice "n" est compris entre 2 et 6, le radical divalent étant choisi de préférence parmi le radial éthylène, propylène, butylène ou méthyl-2 propane-1,2 diyle.

4. Composés selon la revendication 1, **caractérisés par le fait que** l'hétérocycle azoté est choisi parmi la morpholine, la pyrrolidine, la pipérazine, l'homopipérazine et une N-alkyl ($C_1$-$C_6$) pipérazine.

5. Composés selon la revendication 1, **caractérisés par le fait que** les sels sont choisis parmi ceux d'une base minérale ou organique lorsqu'ils comportent une fonction acide carboxylique.

6. Composés selon la revendication 1, **caractérisés par le fait que** les sels sont choisis parmi ceux d'un acide minéral ou organique lorsqu'ils comportent une fonction amine salifiable.

7. Composés selon la revendication 6, **caractérisés par le fait que** les sels sont choisis parmi les halogénures, les nitrates, les sulfates, les sulfonates, les carboxylates, les thiocyanates et les phosphates.

8. Composés selon l'une quelconque des revendications précédentes, **caractérisés par le fait qu'**ils sont choisis dans le groupe constitué par :

le 6-mercapto-8-S-(3-méthylthiopropanoyl) octanoate de méthyle,
le 6-S-(3-méthylthiopropanoyl) 8-mercapto octanoate de méthyle,
le 6-S,8-S-bis (3-méthylthiopropanoyl) octanoate de méthyle,
le 6-mercapto-8-S-(3-méthylthiopropanoyl) octanoate de tertiobutyle,
le 6-S-(3-méthylthiopropanoyl) 8-mercapto octanoate de tertiobutyle,
le 6-S,8-S-bis (3-méthylthiopropanoyl) octanoate de tertiobutyle,
le 6-mercapto-8-S-(3-méthylthiopropanoyl) octanoate de n-butyle,
le 6-S-(3-méthylthiopropanoyl) 8-mercapto octanoate de n-butyle,
le 6-S,8-S-bis (3-méthylthiopropanoyl) octanoate de n-butyle,
l'acide 6-mercapto-8-S(3-méthylthiopropanoyl) octanoïque,
l'acide 6-S-(3-méthylthiopropanoyl) 8-mercapto octanoïque,
l'acide 6-S,8-S-bis(3-méthylthiopropanoyl) octanoïque,
le 6-mercapto-8-S-(3-méthylthiopropanoyl) octanoate d'éthyle,
le 6-S-(3-méthylthiopropanoyl) 8-mercapto octanoate d'éthyle,
le 6-S,8-S-bis (3-méthylthiopropanoyl) octanoate d'éthyle,
le 6-mercapto-8-S-(3-méthylthiopropanoyl) octanoate d'isopropyle,
le 6-S-(3-méthylthiopropanoyl) 8-mercapto octanoate d'isopropyle,
le 6-S,8-S-bis (3-méthylthiopropanoyl) octanoate d'isopropyle,
l'iodure de 6-mercapto-8-S-(3-méthylthiopropanoyl) octanoate de (2' triméthyl ammonium éthyle),
l'iodure de 6-S-(3-méthylthiopropanoyl) 8-mercapto octanoate de (2' triméthyl ammonium éthyle),
l'iodure de 6-S,8-S-bis (3-méthylthiopropanoyl) octanoate de (2' triméthyl ammonium éthyle),
le [6-S,8-S-bis (3-méthylthiopropanoyl) octanoyl]-N-méthyl pipérazine,
le 6-S-acétyl, 8-S-(3-méthylthiopropanoyl) octanoate de méthyle,
le 6-S-(3-méthylthiopropanoyl), 8-S-acétyl octanoate de méthyle,
l'iodure de 6-S-acétyl, 8-S-(3-méthylthiopropanoyl) octanoate de (2' triméthyl ammonium éthyle), et
iodure de 6-S-(3-méthylthiopropanoyl), 8-S-acétyl octanoate de (2' triméthyl ammonium éthyle).

9. Composition pharmaceutique **caractérisée par le fait qu'**elle contient, en une quantité efficace, au moins un composé actif selon l'une quelconque des revendications précédentes dans un excipient pharmaceutiquement acceptable.

**10.** Composition pharmaceutique selon la revendication 9, **caractérisée par le fait qu'**elle contient le composé actif en une quantité efficace pour induire l'apoptose des cellules cancéreuses chez un sujet humain ou animal.

**11.** Composition pharmaceutique selon les revendications 9 et 10, **caractérisée par le fait qu'**elle contient le composé actif en une quantité efficace pour le traitement, la régression et la prévention de cancer chez un sujet humain ou animal.

**12.** Composition pharmaceutique selon les revendications 9 à 11, **caractérisée par le fait qu'**elle se présente sous une forme destinée à une administration par voie parentérale.

**13.** Composition pharmaceutique selon les revendications 9 à 11, **caractérisée par le fait qu'**elle se présente sous une forme destinée à une administration par voie entérale.

**14.** Composition pharmaceutique selon les revendications 9 à 11, **caractérisée par le fait qu'**elle se présente sous une forme destinée à une administration par voie topique.

**15.** Composition pharmaceutique selon l'une quelconque des revendications 9 à 14, **caractérisée par le fait que** le composé actif est présent à une concentration comprise entre 0,1 et 10 % en poids par rapport au poids total de la composition.

**16.** Composition pharmaceutique selon l'une quelconque des revendications 9 à 15, **caractérisée par le fait que** le composé actif est présent à une concentration comprise entre 0,1 et 5 % en poids par rapport au poids total de la composition.

**17.** Composition pharmaceutique selon l'une quelconque des revendications 9 à 16, **caractérisée par le fait qu'**elle est administrée à une dose journalière de 50mg/kg à 100mg/kg de poids corporel.

**18.** Utilisation d'un dérivé de l'acide 6,8-dimercaptooctanoïque substitué en 6-S et/ou 8-S par le radical (3-méthylthiopropanoyl) selon l'une quelconque des revendications 1 à 8, pour la préparation d'une composition pharmaceutique destinée au traitement, à la régression et à la prévention des tumeurs cancéreuses.

**Patentansprüche**

**1.** Derivate der 6,8-Dimercaptooctansäure, in 6-S und/oder 8-S mit dem (3-Methylthiopropanoyl)-Rest substituiert, **dadurch gekennzeichnet, dass** diese der folgenden Formel (I) entsprechen:

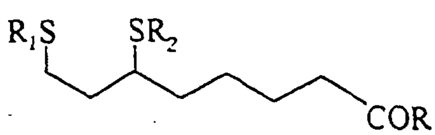

(I)

in der:

R $OR_3$ oder

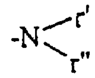

entspricht,

wobei $R_3$ ein Wasserstoffatom, einen linearen oder verzweigten Alkylrest oder einen Rest der Formel

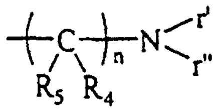

darstellt, n eine ganze Zahl von 1 bis 10 ist und r' und r", die identisch oder unterschiedlich sein können, ein Wasserstoffatom, einen linearen oder verzweigten Alkylrest darstellen, oder zusammen mit dem Stickstoffatom einen stickstoffhaltigen Heterocyclus bilden, der gegebenenfalls mit einem Sauerstoff- oder Stickstoffatom, gegebenenfalls substituiert, substituiert ist,
$R_5$ und $R_4$, die identisch oder unterschiedlich sein können, ein Wasserstoffatom oder einen linearen oder verzweigten Alkylrest darstellen,
$R_1$ und $R_2$, die identisch oder unterschiedlich sein können, ein Wasserstoffatom, einen Rest $COCH_3$ oder einen Rest der folgenden Formel darstellen:

$$-CO(CH_2)_2SCH_3 \quad (II)$$

unter dem Vorbehalt, dass mindestens einer der Reste $R_1$ oder $R_2$ einen Rest der Formel (II) darstellt, und die Enantiomere R und S und deren Racimat sowie die Salze dieser Verbindungen der Formel (I).

2. Verbindungen nach Anspruch 1, **dadurch gekennzeichnet, dass** der lineare oder verzweigte Alkylrest 1 bis 6 Kohlenstoffatome enthält.

3. Verbindungen nach Anspruch 1, **dadurch gekennzeichnet, dass** die Indexzahl "n" 2 bis 6 beträgt, wobei der bivalente Rest bevorzugt unter dem Ethylen-, Propylen, Butylen- oder Methyl-2-propan-1,2-diyl-Rest ausgewählt wird.

4. Verbindungen nach Anspruch 1, **dadurch gekennzeichnet, dass** der stickstoffhaltige Heterocyclus ausgewählt wird unter Morpholin, Pyrrolidin, Piperazin, Homopiperazin und einem N-(C1-C6)-Alkylpiperazin.

5. Verbindungen nach Anspruch 1, **dadurch gekennzeichnet, dass** die Salze ausgewählt werden unter denjenigen einer mineralischen oder organischen Base, falls sie eine Carbonsäurefunktion aufweisen.

6. Verbindungen nach Anspruch 1, **dadurch gekennzeichnet, dass** die Salze ausgewählt werden unter denjenigen einer Mineralsäure oder organischen Säure, falls sie eine salzbildende Aminfunktion aufweisen.

7. Verbindungen nach Anspruch 6, **dadurch gekennzeichnet, dass** die Salze ausgewählt werden unter den Halogeniden, Nitraten, Sulfaten, Sulfonaten, Carboxylaten, Thiocyanaten und Phosphaten.

8. Verbindungen nach einem der vorstehenden Ansprüche, **dadurch gekennzeichnet, dass** diese aus der folgenden Gruppe ausgewählt werden:

6-Mercapto-8-S-(3-methylthiopropanoyl)-methyloctanoat,
6-S-(3-Methylthiopropanoyl)-8-mercaptomethyloctanoat,
6-S,8-S-bis(3-Methylthiopropanoyl)-methyloctanoat,
6-Mercapto-8-S-(3-methylthiopropanoyl)-tert-butyloctanoat,
6-S-(3-Methylthiopropanoyl)-8-mercapto-tert-butyloctanoat,
6-S, 8-S-bis(3-Methylthiopropanoyl)-tert-butyloctanoat,
6-Mercapto-8-S-(3-methylthiopropanoyl)-n-butyloctanoat,
6-S-(3-Methylthiopropanoyl)-8-mercapto-n-butyloctanoat,
6-S, 8-S-bis(3-Methylthiopropanoyl)-n-butyloctanoat,
6-Mercapto-8-S-(3-methylthiopropanoyl)-octansäure,
6-S-(3-Methylthiopropanoyl)-8-mercaptooctansäure,
6-S,8-S-bis(3-Methylthiopropanoyl)-octansäure,
6-Mercapto-8-S-(3-methylthiopropanoyl)-ethyloctanoat,
6-S-(3-Methylthiopropanoyl)-8-mercaptoethyloctanoat,

6-S,8-S-bis(3-Methylthiopropanoyl)-ethyloctanoat,
6-Mercapto-8-S-(3-methylthiopropanoyl)-isopropyloctanoat,
6-S-(3-Methylthiopropanoyl)-8-mercaptoisopropyloctanoat,
6-S,8-S-bis(3-Methylthiopropanoyl)-isopropyloctanoat,
6-Mercapto-8-S-(3-methylthiopropanoyl)-(2'-trimethylammoniumethyl)-octanoatjodid,
6-S-(3-Methylthiopropanoyl)-8-mercapto-(2'-trimethylammoniumethyl)-octanoatjodid,
6-S, 8-S-bis(3-Methylthiopropanoyl)-(2'-trimethylammoniumethyl)-octanoatjodid,
[6-S,8-S-bis(3-Methylthiopropanoyl)-octanoyl]-N-methylpiperazin,
6-S-Acetyl-8-S-(3-methylthiopropanoyl)-methyloctanoat,
6-S-(3-Methylthiopropanoyl)-8-S-acetylmethyloctanoat,
6-S-Acetyl-8-S-(3-methylthiopropanoyl)-(2'-trimethylammoniumethyl)-octanoatjodid, und
6-S-(3-Methylthiopropanoyl)-8-S-acetyl-(2'-trimethylammoniumethyl)octanoatjodid.

**9.** Pharmazeutische Zubereitung, **dadurch gekennzeichnet, dass** diese in einer ausreichenden Menge mindestens einen Wirkstoff nach einem der vorstehenden Ansprüche in einem pharmazeutisch annehmbaren Arzneimittelträger enthält.

**10.** Pharmazeutische Zubereitung nach Anspruch 9, **dadurch gekennzeichnet, dass** diese den Wirkstoff in einer ausreichenden Menge enthält, um die Apoptose von Krebszellen bei einem Menschen oder einem Tier zu induzieren.

**11.** Pharmazeutische Zubereitung nach den Ansprüchen 9 und 10, **dadurch gekennzeichnet, dass** diese den Wirkstoff in einer ausreichenden Menge für die Behandlung, den Rückgang und die Prävention von Krebs bei einem Menschen oder einem Tier enthält.

**12.** Pharmazeutische Zubereitung nach den Ansprüchen 9 bis 11, **dadurch gekennzeichnet, dass** diese in einer Form vorliegt, die für eine parenterale Verabreichung bestimmt ist.

**13.** Pharmazeutische Zubereitung nach den Ansprüchen 9 bis 11, **dadurch gekennzeichnet, dass** diese in einer Form vorliegt, die für eine enterale Verabreichung bestimmt ist.

**14.** Pharmazeutische Zubereitung nach den Ansprüchen 9 bis 11, **dadurch gekennzeichnet, dass** diese in einer Form vorliegt, die für eine topische Verabreichung bestimmt ist.

**15.** Pharmazeutische Zubereitung nach einem der Ansprüche 9 bis 14, **dadurch gekennzeichnet, dass** der Wirkstoff in einer Konzentration von 0,1 bis 10 Gew.-%, bezogen auf das Gesamtgewicht der Zubereitung, vorliegt.

**16.** Pharmazeutische Zubereitung nach einem der Ansprüche 9 bis 15, **dadurch gekennzeichnet, dass** der Wirkstoff in einer Konzentration von 0,1 bis 5 Gew.-%, bezogen auf das Gesamtgewicht der Zubereitung, vorliegt.

**17.** Pharmazeutische Zubereitung nach einem der Ansprüche 9 bis 16, **dadurch gekennzeichnet, dass** diese in einer täglichen Dosis von 50 mg/kg Körpergewicht bis 100 mg/kg Körpergewicht verabreicht wird.

**18.** Verwendung eines Derivats der 6,8-Dimercaptooctansäure, in 6-S und/oder 8-S mit dem (3-Methylthiopropanoyl)-Rest substituiert, nach einem der Ansprüche 1 bis 8 für die Herstellung einer pharmazeutischen Zubereitung, die für die Behandlung, den Rückgang und die Prävention von krebsartigen Tumoren bestimmt ist.

## Claims

**1.** 6,8-Dimercaptooctanoic acid derivatives substituted at the 6-S and/or 8-S position with the (3-methylthiopropanoyl) radical, **characterized in that** they correspond to the following formula (I):

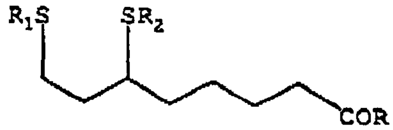

(I)

in which:

R represents $OR_3$ or

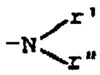

$R_3$ representing a hydrogen atom, a linear or branched alkyl radical , or a radical of formula

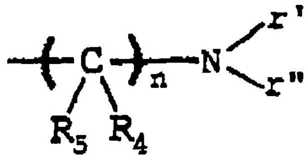

,

n being an integer from 1 to 10, and r' and r", which are identical or different, representing a hydrogen atom, a linear or branched alkyl radical or, taken together form, with the nitrogen atom, a nitrogen-containing heterocycle optionally substituted with an optionally substituted oxygen or nitrogen atom,

$R_5$ and $R_4$, which are identical or different, represent a hydrogen atom or a linear or branched alkyl radical,

$R_1$ and $R_2$, which are identical or different, represent a hydrogen, a radical $-COCH_3$ atom or a radical of formula:

$$-CO(CH_2)_2SCH_3 \quad (II)$$

with the proviso that at least one of the radicals $R_1$ or $R_2$ represents a radical of formula (II), and the R and S enantiomers and their racemate as well as the salts of the said compounds of formula (I).

**2.** Compounds according to Claim 1, **characterized in that** the linear or branched alkyl radical contains from 1 to 6 carbon atoms.

**3.** Compounds according to Claim 1, **characterized in that** the subscript "n" is between 2 and 6, the divalent radical being preferably chosen from the ethylene, propylene, butylene or 2-methyl-1,2-propanediyl radical.

**4.** Compounds according to Claim 1, **characterized in that** the nitrogen-containing heterocycle is chosen from morpholine, pyrrolidine, piperazine, homopiperazine and an N-($C_1$-$C_6$)alkylpiperazine.

**5.** Compounds according to Claim 1, **characterized in that** the salts are chosen from those of an inorganic or organic base when they comprise a carboxylic acid functional group.

**6.** Compounds according to Claim 1, **characterized in that** the salts are chosen from those of an inorganic or organic acid when they comprise a salifiable amine functional group.

**7.** Compounds according to Claim 6, **characterized in that** the salts are chosen from halides, nitrates, sulphates, sulphonates, carboxylates, thiocyanates and phosphates.

**8.** Compounds according to any one of the preceding claims, **characterized in that** they are chosen from the group consisting of:

methyl 6-mercapto-8-S-(3-methylthiopropanoyl)octanoate,
methyl 6-S-(3-methylthiopropanoyl)-8-mercaptooctanoate,
methyl 6-S,8-S-bis(3-methylthiopropanoyl)octanoate,
tert-butyl 6-mercapto-8-S-(3-methylthiopropanoyl)-octanoate,
tert-butyl 6-S-(3-methylthiopropanoyl) -8-mercaptooctanoate,
tert-butyl 6-S,8-S-bis(3-methylthiopropanoyl)octanoate, n-butyl 6-mercapto-8-S-(3-methylthiopropanoyl)-octanoate,
n-butyl 6-S-(3-methylthiopropanoyl)-8-mercaptooctanoate,
n-butyl 6-S,8-S-bis(3-methylthiopropanoyl)octanoate,
6-mercapto-8-S-(3-methylthiopropanoyl)octanoic acid,
6-S-(3-methylthiopropanoyl)8-mercaptooctanoic acid,
6-S,8-S-bis(3-methylthiopropanoyl)octanoic acid,
ethyl 6-mercapto-8-S-(3-methylthiopropanoyl)octanoate,
ethyl 6-S-(3-methylthiopropanoyl)-8-mercaptooctanoate,
ethyl 6-S,8-S-bis(3-methylthiopropanoyl)octanoate,
isopropyl 6-mercapto-8-S-(3-methylthiopropanoyl)-octanoate,
isopropyl 6-S-(3-methylthiopropanoyl)-8-mercaptooctanoate,
isopropyl 6-S,8-S-bis(3-methylthiopropanoyl)octanoate,
(2'-trimethylammoniumethyl) 6-mercapto-8-S-(3-methyl-thiopropanoyl)octanoate iodide,
(2'-trimethylammoniumethyl) 6-S- (3-methylthiopropanoyl)-8-mercaptooctanoate iodide,
(2'-trimethylammoniumethyl) 6-S,8-S-bis(3-methylthiopropanoyl)octanoate iodide,
[6-S,8-S-bis(3-methylthiopropanoyl)octanoyl]N-methylpiperazine,
methyl 6-S-acetyl-8-S-(3-methylthiopropanoyl)octanoate, methyl 6-S-(3-methylthiopropanoyl)-8-S-acetyloctanoate, (2'-trimethylammoniumethyl) 6-S-acetyl-8-S-(3-methylthiopropanoyl)octanoate iodide, and
(2'-trimethylammoniumethyl) 6-S-(3-methylthiopropanoyl)-8-S-acetyloctanoate iodide.

**9.** Pharmaceutical composition **characterized in that** it contains, in an effective quantity, at least one active compound according to any one of the preceding claims in a pharmaceutically acceptable excipient.

**10.** Pharmaceutical composition according to Claim 9, **characterized in that** it contains the active compound in an effective quantity to induce apoptosis of cancer cells in a human or animal subject.

**11.** Pharmaceutical composition according to Claims 9 and 10, **characterized in that** it contains the active compound in an effective quantity for the treatment, regression and prevention of cancer in a human or animal subject.

**12.** Pharmaceutical composition according to Claims 9 to 11, **characterized in that** it is provided in a form intended for administration by the parenteral route.

**13.** Pharmaceutical composition according to Claims 9 to 11, **characterized in that** it is provided in a form intended for administration by the enteral route.

**14.** Pharmaceutical composition according to Claims 9 to 11, **characterized in that** it is provided in a form intended for administration by the topical route.

**15.** Pharmaceutical composition according to any one of Claims 9 to 14, **characterized in that** the active compound is present in a concentration of between 0.1 and 10% by weight relative to the total weight of the composition.

**16.** Pharmaceutical composition according to any one of Claims 9 to 15, **characterized in that** the active compound is present in a concentration of between 0.1 and 5% by weight relative to the total weight of the composition.

**17.** Pharmaceutical composition according to any one of Claims 9 to 16, **characterized in that** it is administered at a daily dose of 50 mg/kg to 100 mg/kg of bodyweight.

**18.** Use of a 6,8-dimercaptooctanoic acid derivative substituted at the 6-S and/or 8-S position with the (3-methylthiopropanoyl) radical according to any one of Claims 1 to 8, for the preparation of a pharmaceutical composition intended for the treatment, regression and prevention of cancerous tumours.

**FIGURE 1**

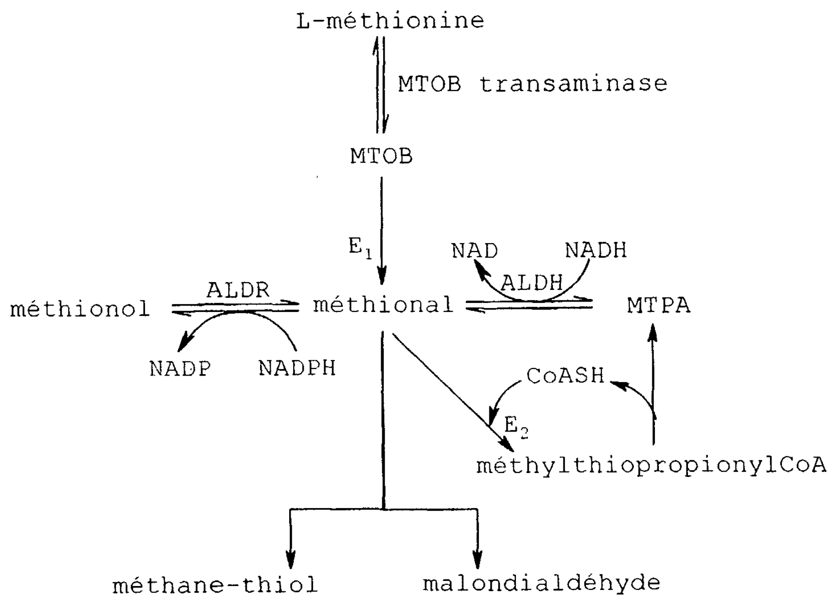


- MTOB représente l'acide 4-méthylthio-2-oxobutanoïque,
- MTPA représente l'acide méthylthiopropionique,
- $E_1$ représente la décarboxylase du complexe déshydrogénase oxo-acide à chaîne branchée dont le co-facteur est la thiamine pyrophosphate,
- $E_2$ représente la transacylase du complexe déshydrogénase oxo-acide à chaîne branchée dont le co-facteur est l'acide thioctique,
- ALDR représente l'aldéhyde réductase,
- ALDH représente l'aldéhyde déshydrogénase,
- CoASH représente l'acétyl coenzyme A,
- NADH/NAD représente le nicotinamide adénine dinucléotide, et
- NADPH/NADP représente le nicotinamide adénine dinucléotide phosphate.

**FIGURE 2**

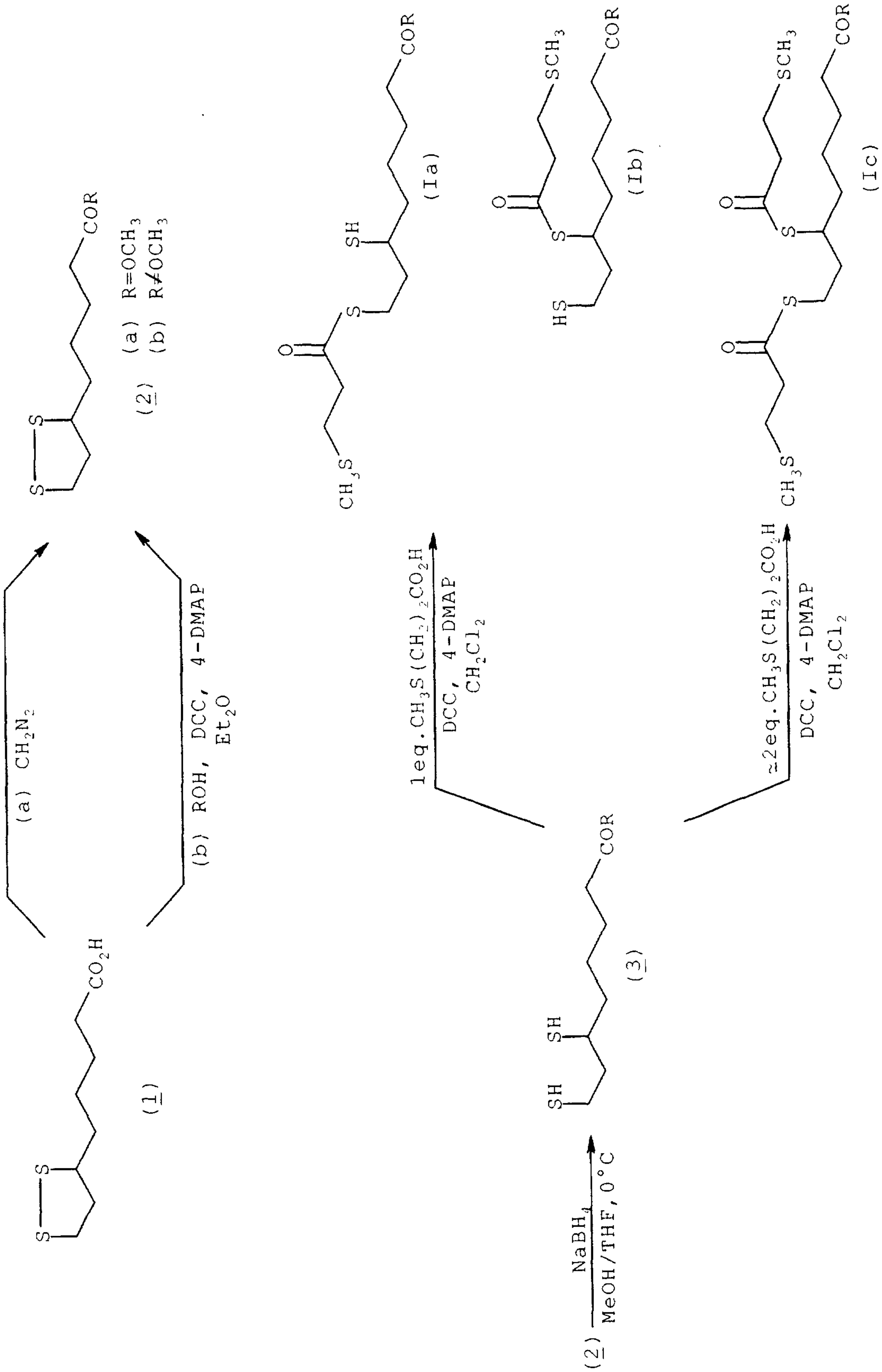


(1) (2) (3) (Ia) (Ib) (Ic)

FIGURE 3

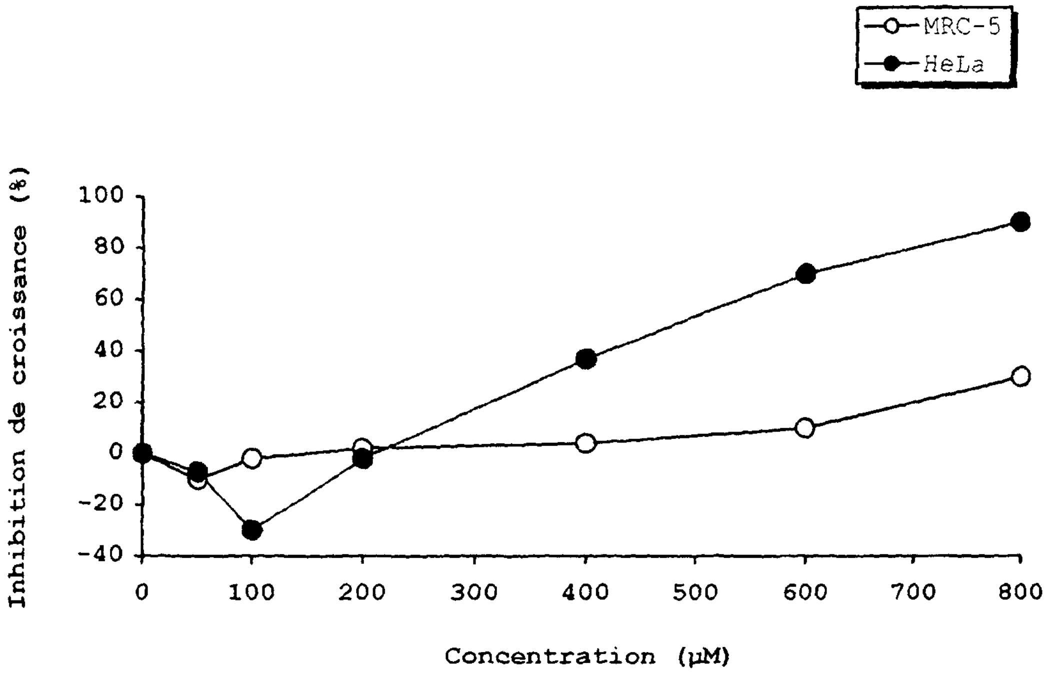


FIGURE 4

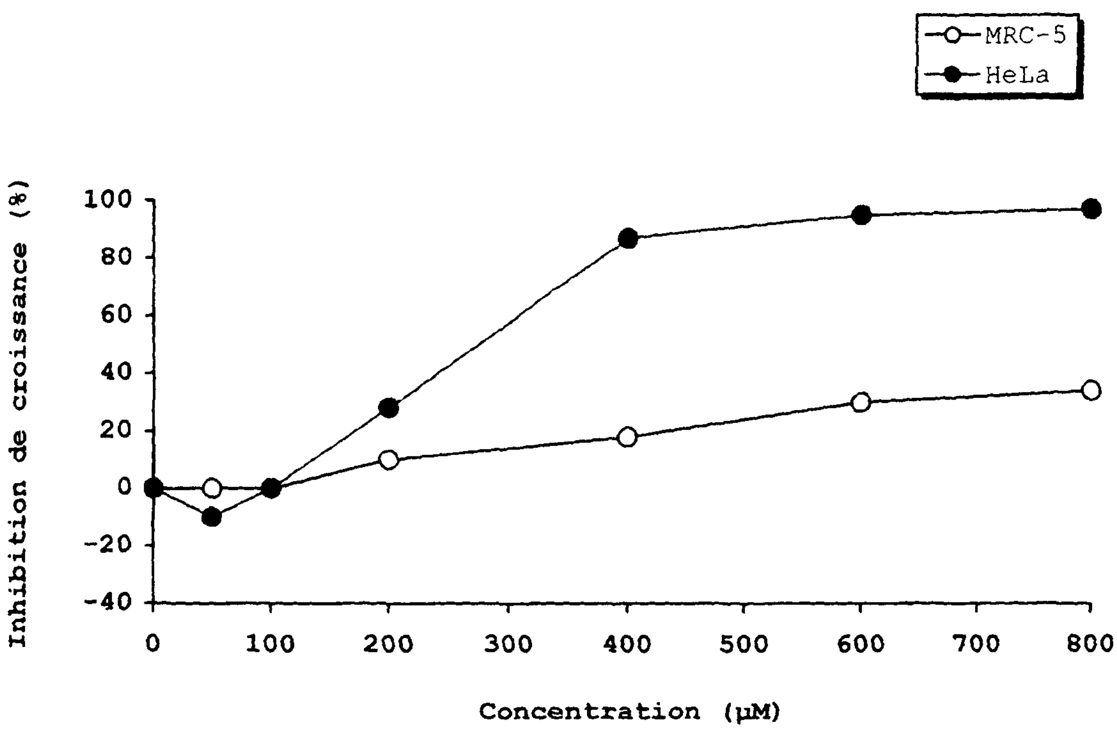

FIGURE 5

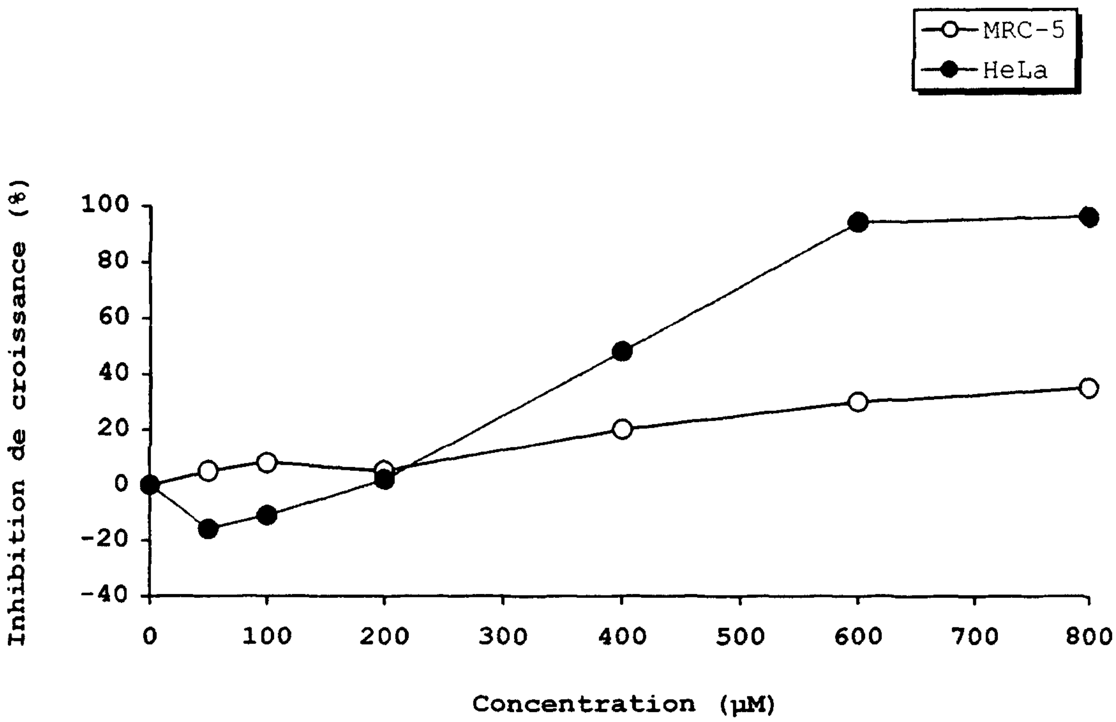


FIGURE 6

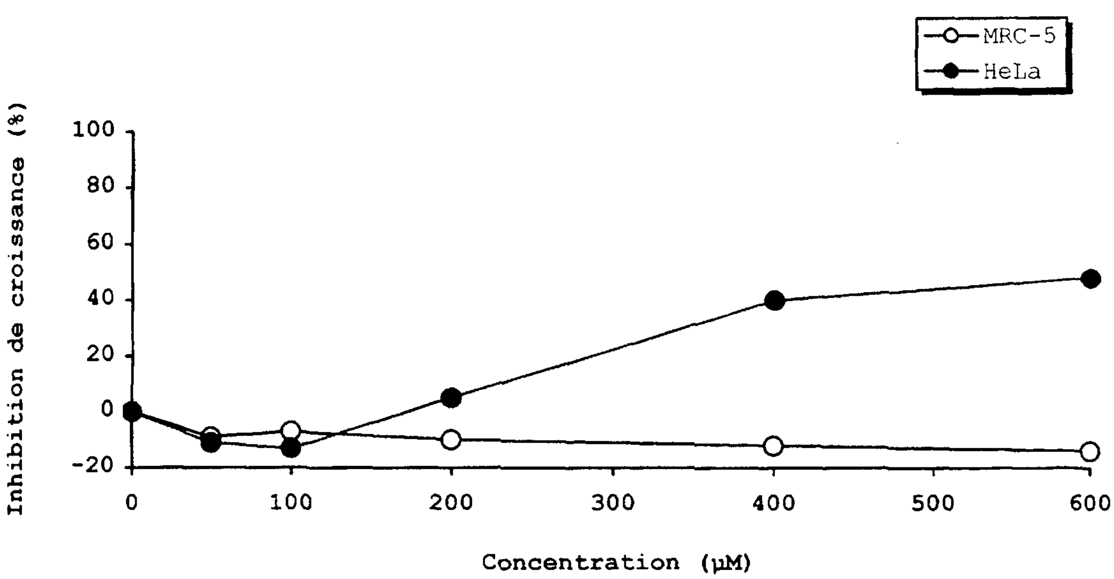

FIGURE 7

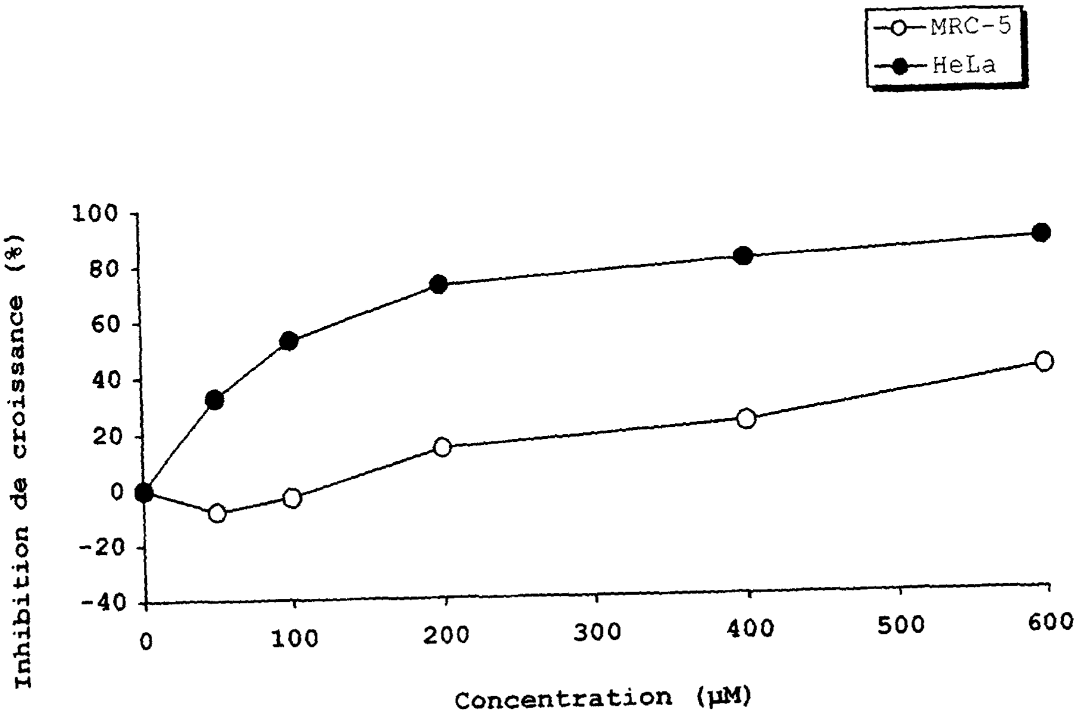


FIGURE 8

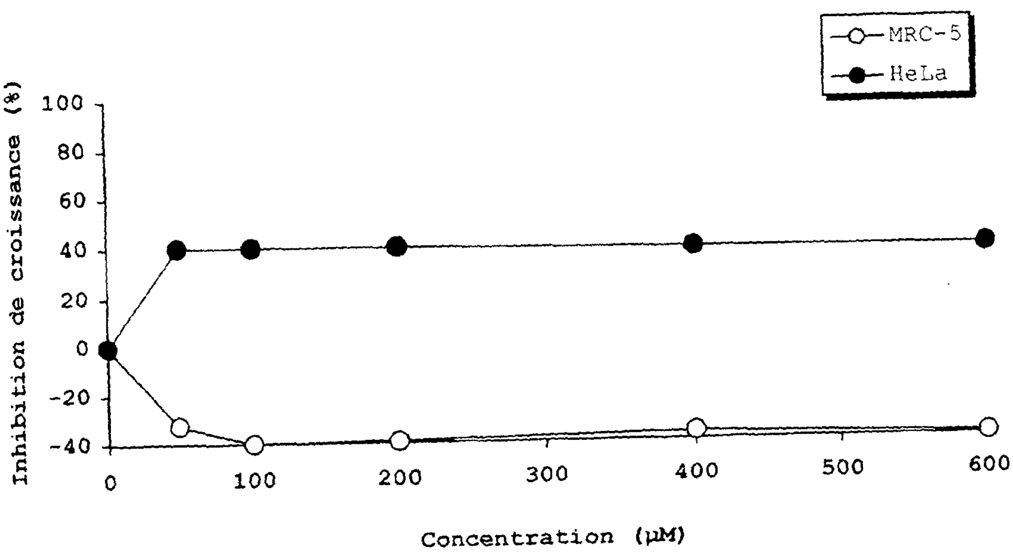

FIGURE 9

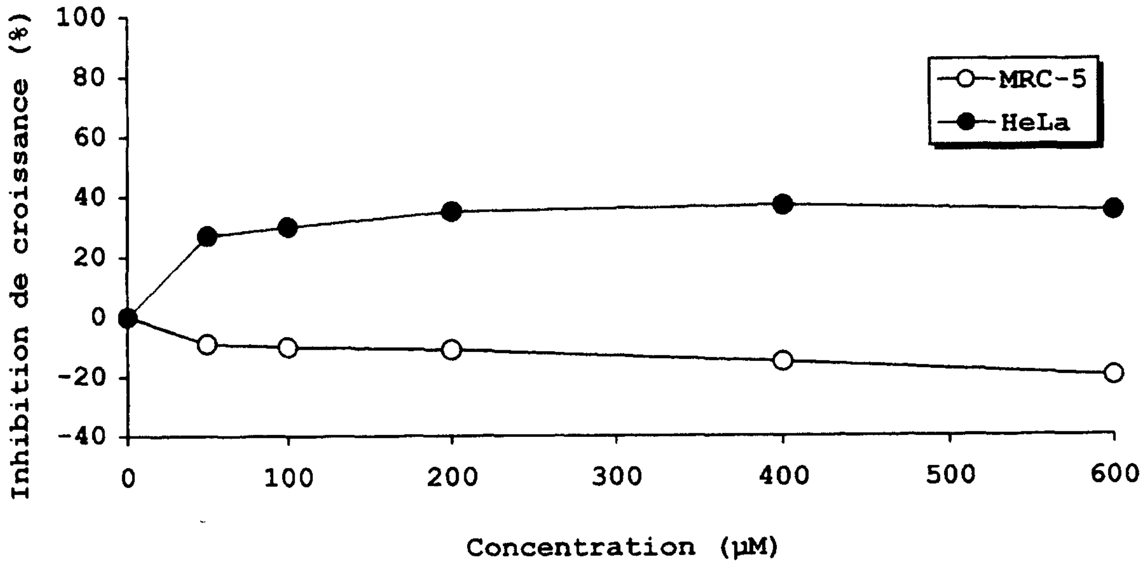


FIGURE 10

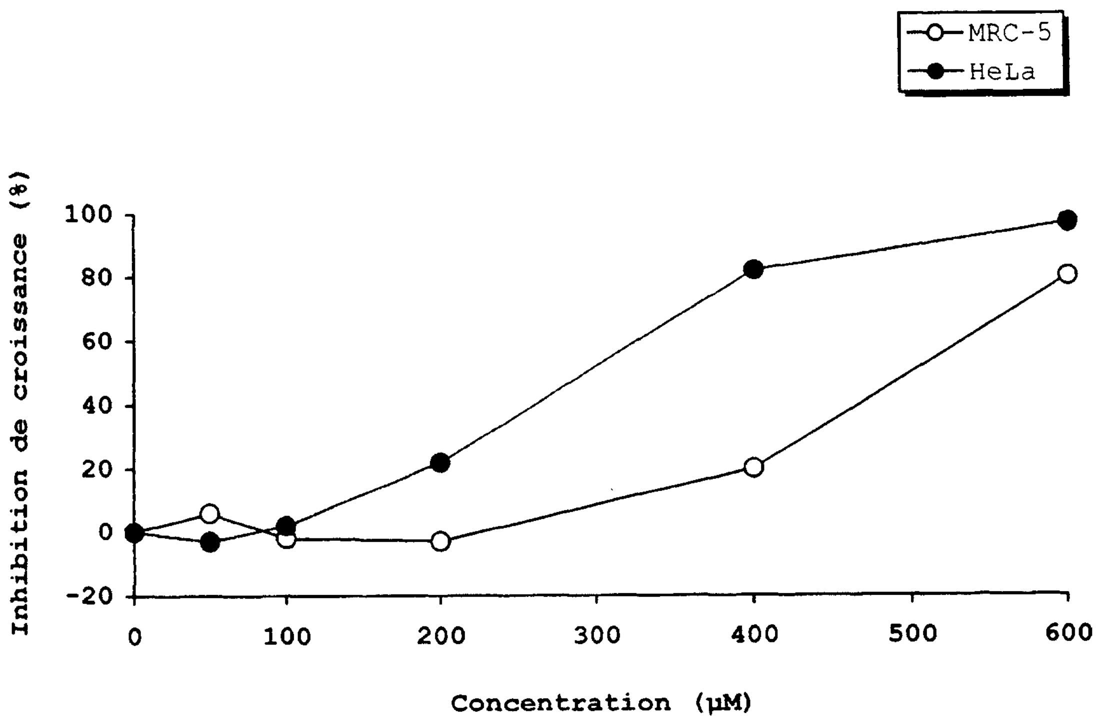

FIGURE 11

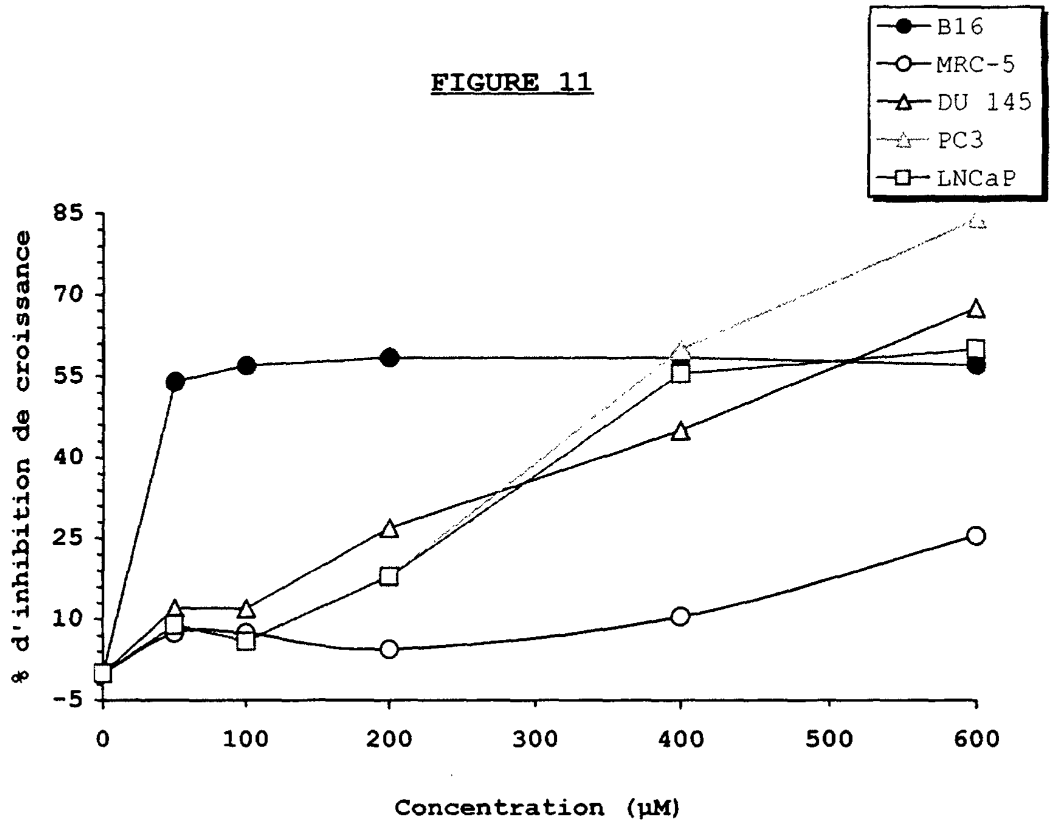


FIGURE 12

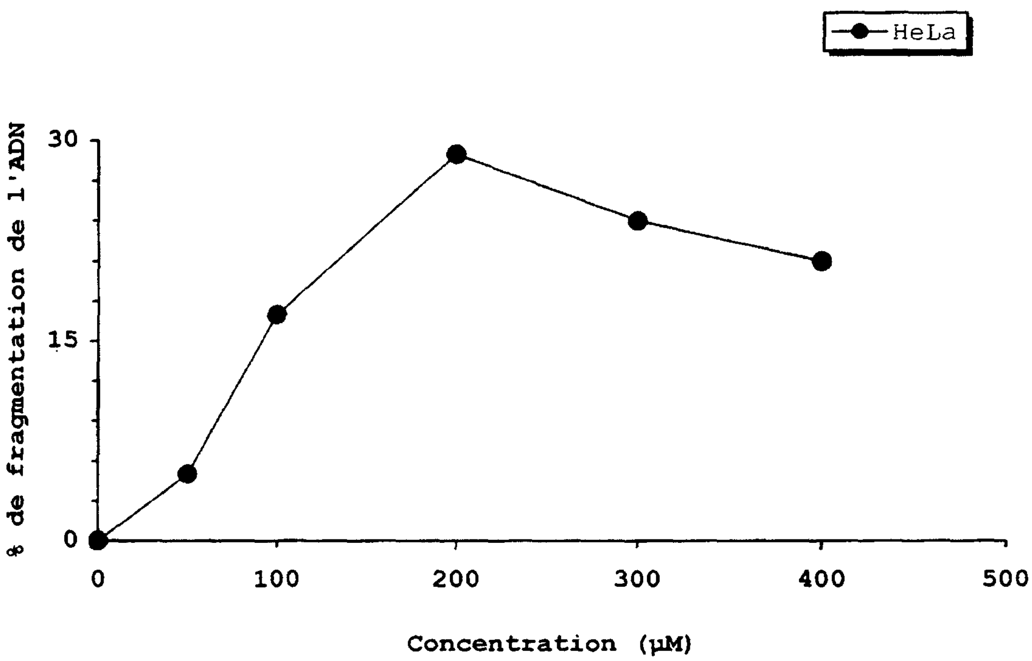

FIGURE 13

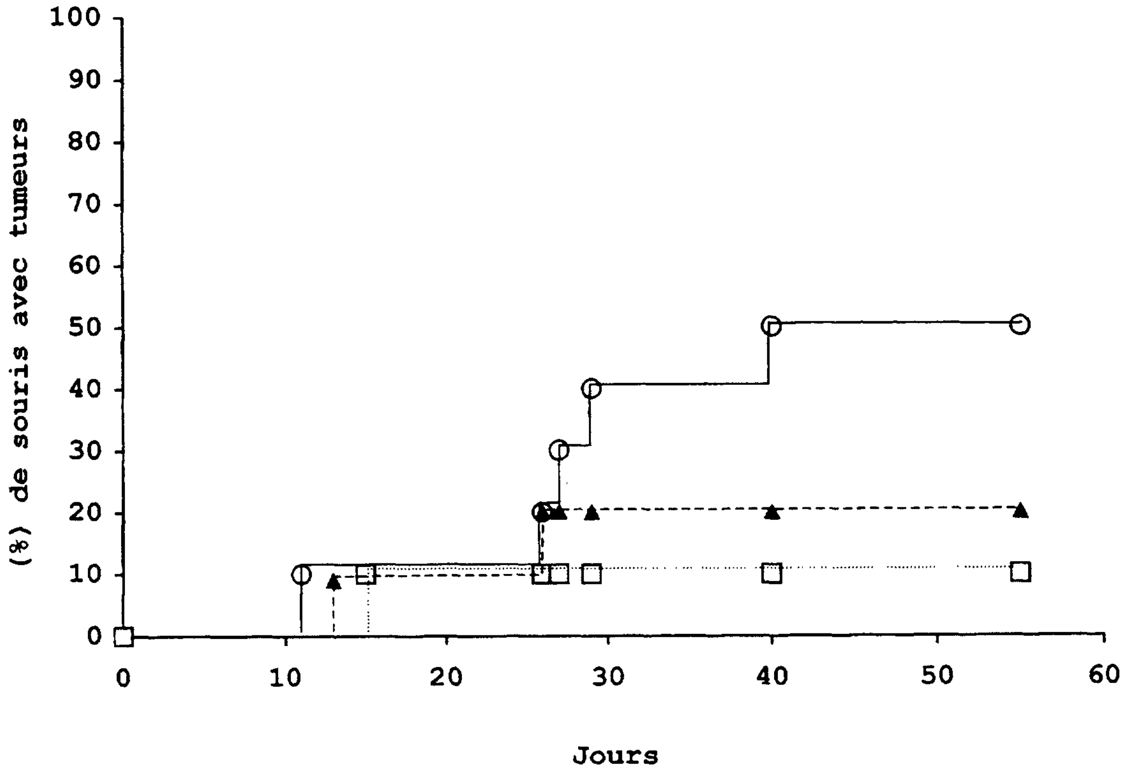
100
90
80
70
60
50
40
30
20
10
0
(%) de souris avec tumeurs
0
10
20
30
40
50
60
Jours